(19) Europäisches Patentamt
European Patent Office
Office européen des brevets

(11) EP 0 407 137 B1

(12) **EUROPEAN PATENT SPECIFICATION**

(45) Date of publication and mention of the grant of the patent: **01.05.1996 Bulletin 1996/18**

(21) Application number: **90307251.0**

(22) Date of filing: **03.07.1990**

(51) Int. Cl.$^6$: **C07D 453/02**, C07D 265/36, C07D 279/16, C07D 451/04, C07D 451/14, C07D 413/12, C07D 417/12, A61K 31/535, A61K 31/54

# (54) Benzazine compounds and pharmaceutical uses thereof

Benzazin-Verbindungen und deren pharmazeutische Verwendungen

Dérivés de benzazine et leurs applications pharmaceutiques

(84) Designated Contracting States:
**AT BE CH DE DK ES FR GB IT LI LU NL SE**

(30) Priority: **03.07.1989 JP 171549/89**
**12.03.1990 JP 60663/90**

(43) Date of publication of application:
**09.01.1991 Bulletin 1991/02**

(73) Proprietor: **YOSHITOMI PHARMACEUTICAL INDUSTRIES, LTD.**
**Osaka-shi Osaka 541 (JP)**

(72) Inventors:
- **Kawakita, Takeshi**
**Nakatsu-shi, Oita 871 (JP)**
- **Kuroita, Takanobu**
**Nakatsu-shi, Oita 871 (JP)**
- **Fukuda, Takemi**
**Kobe-shi, Hyogo 658 (JP)**
- **Ikezawa, Ryuhei**
**Oita 871 (JP)**

(74) Representative: **Hardisty, David Robert et al**
**BOULT, WADE & TENNANT**
**27 Furnival Street**
**London EC4A IPQ (GB)**

(56) References cited:
**EP-A- 0 234 872** **EP-A- 0 313 393**
**DE-A- 2 509 155**

Remarks:
The file contains technical information submitted after the application was filed and not included in this specification

EP 0 407 137 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

It has been ascertained that 5-$HT_3$ receptors, which are of one class of the subtypes of the serotonin (5-HT) receptors, are not only present in the sensory nervous system and the autonomic nervous system, but distributed in the central nervous system. As the whole aspect of the 5-$HT_3$ receptors is being revealed, it has been suggested that the clinical application of antagonistic drugs for the receptors can range widely from the peripheries to the center.

It has been known that the compounds showing an antagonistic activity for 5-$HT_3$ receptors are useful for the treatment of disturbances in central nervous systems such as depression, anxiety, psychopathy and dementia, for the prophylaxis or treatment of digestive diseases such as indigestion, nausea, vomiting, diarrhea, indefinite complaint of alimentary system, irritable colon syndrome, and/or for the relief or treatment of dependance induced by drug abuse. As such 5-$HT_3$ receptor-blocking agent, ICS 205-930 ((3$\alpha$-tropanyl)-1H-indole-3-carboxylic acid ester), Ondansetron (1,2,3,9-tetrahydro-9-methyl-3[(2-methyl-1H-imidazol-1-yl)methyl]-4H-carbazole-4-one) and the like are known. Further, U.S. Patent 4,892,872 discloses 3-oxo-2H-1,4-benzoxazine-8-carboxamide compounds which shows an antagonistic activity for 5-$HT_3$ receptors. Further, a certain class of benzamides have a dopermine $D_2$ receptor-blocking activity and are useful as drugs for the treatment of psychopathy or as anti-vomiting drugs. For example, Sulpiride (2-methoxy-N-(1-ethyl-2-pyrrolidinylmethyl)-5-sulfamoylbenzamide) is being used as a drug for the treatment of psychopathy or peptic ulcer, while Metoclopramide (4-amino-5-chloro-N-(2-diethylamine)ethyl-2-methoxybenzamide) is being used as drugs for regulating enterokinesis or as anti-vomiting drugs.

As a result of studies made by the present inventors, a series of novel compound having a different structure from those of the above-mentioned compounds and having a 5-$HT_3$ receptor-blocking activity and/or dopamine $D_2$ receptor-blocking activity was found.

The present invention provides a benzazine compound, its geometrical isomer and optical isomer, and a pharmaceutically acceptable salt thereof, all of which are novel compounds that are pharmacologically active and useful as medicines and an intermediate thereof and pharmaceutical uses of said benzazine compounds.

The present invention provides a benzazine compound, a geometrical isomer of said benzazine compound, an optical isomer of said benzazine compound, and a pharmaceutically acceptable salt of said benzazine compound, said benzazine compound being represented by formula (I):

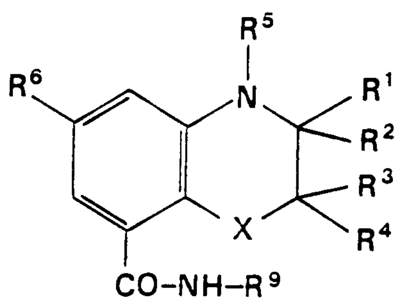

(I)

(wherein $R^1$, $R^2$, $R^3$, and $R^4$ are the same or different and each represents hydrogen, an alkyl, phenyl, a substituted phenyl, a phenylalkyl, a substituted phenylalkyl, a heteroaryl, a substituted heteroaryl, a heteroarylalkyl, or a substituted heteroarylalkyl, or one of $R^1$ and $R^2$ is bonded to one of $R^3$ and $R^4$ to form a single bond; $R^5$ represents hydrogen, an alkyl, an alkanoyl, an aroyl, a substituted aroyl, a heteroaroyl, a substituted heteroaroyl, a phenylalkyl, a substituted phenylalkyl, carbamoyl, a mono- or di-substituted carbamoyl, an aminoalkyl, or a mono- or di-substituted aminoalkyl, or $R^5$ may be bonded to one of $R^1$ and $R^2$ to form a single bond; $R^6$, $R^7$, and $R^8$ are the same or different and each represents hydrogen, a halogen, an alkyl, an alkoxy, amino, an acylamino, a mono- or dialkyl-substituted amino, hydroxyl group, a protected hydroxyl group, nitro, a haloalkyl, cyano, $-S(O)_\ell R^{18}$ ($R^{18}$ represents an alkyl, phenyl or a substituted phenyl and $\ell$ is 0, 1 or 2) or

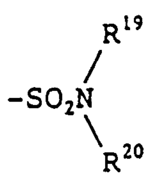

($R^{19}$ and $R^{20}$ are the same or different and each represents hydrogen or an alkyl); X represents oxygen or sulfur; Y represents oxygen or N-$R^{10}$ ($R^{10}$ represents hydrogen or an alkyl); and $R^9$ represents the formula:

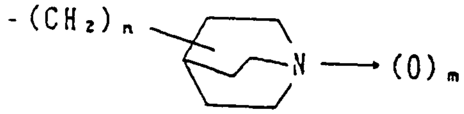

(m is 0 or 1 and n is 0, 1, or 2),

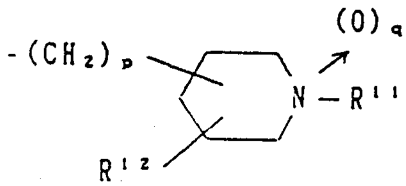

(p is 0, 1, or 2, q is 0 or 1, $R^{11}$ represents an alkyl, a phenylalkyl, a substituted phenylalkyl, a phenoxyalkyl, or a substituted phenoxyalkyl, and $R^{12}$ represents hydrogen or an alkoxy),

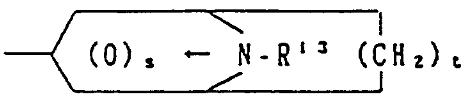

(s and t each is 0 or 1 and $R^{13}$ represents an alkyl, a phenylalkyl, or a substituted phenylalkyl),

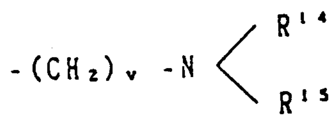

($R^{14}$ and $R^{15}$ are the same or different and each represents hydrogen, an alkyl, a phenylalkyl, or a substituted phenylalkyl, or $R^{14}$ and $R^{15}$ are bonded to each other to be a group which forms a heterocyclic ring in cooperation with the adjacent nitrogen atom, and v is an integer of 1 to 8), or

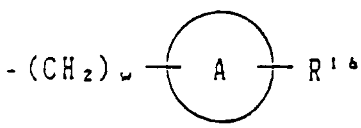

(ring A represents a condensed or uncondensed, nitrogen-containing heterocyclic ring group which may further contain oxygen, sulfur, or N-$R^{17}$ ($R^{17}$ represents hydrogen or an alkyl) in the ring thereof, $R^{16}$ represents hydrogen, an alkyl, an alkenyl, an alkynyl, a phenylalkyl, a substituted phenylalkyl, amino, a mono- or dialkyl-substituted amino, or an acylamino, and w is 1, 2, 3, or 4)), as further defined in the claims.

The present invention further provides a compound or a reactive derivative on a carboxyl group thereof, said compound being represented by formula (II):

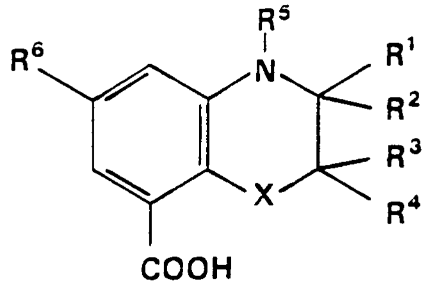

(II)

(wherein each symbol has the same meaning as that defined above).

The present invention further provides a pharmaceutical composition comprising the benzazine compound of formula (I) above, an isomer thereof or a pharmaceutically acceptable salt thereof.

The above definitions are explained below in more detail. The halogen can be fluorine, chlorine, bromine, or iodine; the alkyl can be one having 1 to 20 carbon atoms, such as methyl, ethyl, propyl, isopropyl, butyl, isobutyl, tertiary butyl, pentyl, hexyl, heptyl, octyl, nonyl, decyl, dodecyl, tetradecyl, octadecyl or icosyl; the alkenyl can be one having 2 to 20 carbon atoms, such as vinyl, allyl, 1-propenyl, butenyl or hexenyl; the alkynyl can be one having 2 to 20 carbon atoms, such as ethynyl, 2-propynyl, butynyl, 4-pentynyl or hexynyl; the alkoxy can be one having 1 to 8 carbon atoms, such as methoxy, ethoxy, propoxy, isopropoxy, butoxy, isobutoxy, tertiary butoxy, pentyloxy, hexyloxy, heptyloxy or octyloxy; the phenylalkyl can be benzyl, 1- or 2-phenylethyl, 3-phenylpropyl or 4-phenylbutyl; the heteroaryl can be pyridyl, thienyl, furyl, imidazolyl, pyrazolyl or pyrimidinyl; the heteroarylalkyl can be one in which the heteroaryl moiety means the heteroaryl as mentioned above and the alkyl moiety has 1 to 8 carbon atoms, such as, for example, pyridylmethyl, thienylmethyl, furylmethyl or imidazolylmethyl; the alkanoyl can be one having 2 to 5 carbon atoms such as acetyl, propionyl, butyryl, isobutyryl, valeryl, isovaleryl, or the like; the aroyl can be benzoyl, naphthoyl, phenylacetyl, phenylpropionyl or phenylbutyryl; the heteroaroyl can be 2- or 3-furoyl, 2- or 3-thenoyl, nicotinoyl or isonicotinoyl; the mono-or di-substituted carbamoyl can be the substituted carbanoyl substituted by 1 or 2 alkyls having 1 to 8 carbon atoms such as N-methylcarbamoyl, N-ethylcarbamoyl, N-propylcarbamoyl, N-isopropylcarbamoyl, N-butylcarbamoyl, N-hexylcarbamoyl, N-octylcarbamoyl, N,N-dimethylcarbamoyl, N,N-diethylcarbamoyl, N,N-dipropylcarbamoyl, N,N-diisopropylcarbamoyl, N,N-dibutylcarbamoyl, N,N-dihexylcarbamoyl or N,N-dioctylcarbamoyl; the aminoalkyl can be one in which the alkyl moiety has 1 to 8 carbon atoms, such as, for example, aminomethyl, 2-aminoethyl, 3-aminopropyl, 4-aminobutyl, 5-aminopentyl or 5-aminohexyl; the mono- or di-substituted aminoalkyl can be one in which the amino moiety has been mono- or di-substituted and the alkyl moiety has 1 to 8 carbon atoms, such as, for example, methylaminomethyl, dimethylaminomethyl, 2-methylaminoethyl, 2-dimethylaminoethyl, 3-methylaminopropyl or 3-dimethylaminopropyl; the acylamino can be an alkanoylamino having 2 to 5 carbon atoms, such as acetylamino, propionylamino, butyrylamino or pivaloylamino; the mono- or dialkyl-substituted amino can be one in which the alkyl moiety has 1 to 8 carbon atoms, such as, for example, methylamino, ethylamino, propylamino, isopropylamino, butylamino, hexylamino, octylamino, dimethylamino, diethylamino, dipropylamino, diisopropylamino, dibutylamino, dihexylamino or dioctylamino; the protected hydroxyl group can be hydroxyl group protected with, for example, benzyl, methoxymethyl, methylthiomethyl, ethoxyethyl, benzyloxymethyl or pyranyl; the haloalkyl can be one having 1 to 4 carbon atoms, such as trifluoromethyl, 2,2,2-trifluoroethyl, 2,3,3-trifluoropropyl, 1,1,2,2-tetrafluoroethyl or 2,2,3,3-tetrafluoropropyl; and the phenoxyalkyl can be phenoxymethyl, 2-phenoxyethyl, 3-phenoxypropyl or 4-phenoxybutyl. The group which forms a heterocyclic ring in cooperation with the adjacent nitrogen atom can be a cyclic amino group which may contain, besides the nitrogen atom, a hetero atom such as nitrogen, oxygen or sulfur, and examples of such group include 5- to 7-membered saturated cyclic amino groups such as pyrrolidinyl, morpholino, thiomorpholino, piperidino, piperazinyl or homopiperazinyl. This cyclic amino group may contain a substituent group at a position where substitution is possible, and examples of such substituent group include an alkyl, phenyl, a substituted phenyl, a phenylalkyl, a substituted phenylalkyl or an alkanoyl. The condensed or uncondensed, nitrogen-containing heterocyclic ring which may further contain oxygen, sulfur, or N-$R^{17}$ ($R^{17}$ represents hydrogen or an alkyl) in the ring thereof can be 2- or 3-pyrrolidinyl, morpholinyl, thiomorpholinyl, piperidyl, thiazolyl, isothiazolyl, oxazolyl, isoxazolyl, pyrazolyl, imidazolyl, 1-methyl-2-imidazolyl, pyridazinyl, pyrimidinyl, 1H-indazolyl, purinyl, quinoxalinyl, phthalazinyl, quinazolinyl, cinnolinyl, pteridinyl, benzothiazolyl, 1,2-benzisothiazolyl, benzoxazolyl, 1,2-benzisoxazolyl, benzimidazolyl or 1-methyl-2-benzimidazolyl. The substituent group(s) contained in the substituted phenyl, substituted phenylalkyl, substituted phenoxyalkyl, substituted heteroaryl, substituted heteroarylalkyl, substituted aroyl or substituted heteroaroyl can be 1 to 3 substituents selected from halogens, alkoxys, alkyls, nitro,

amino, haloalkyl, carboxy, and alkoxycarbonyls (these halogens, alkyls, haloalkyls and alkoxys are the same as defined above).

Among compounds of the present invention, compounds of formula (I) wherein Y represents NH are preferred. Among them, 6-chloro-4-methyl-N-(3-quinuclidinyl)-3,4-dihydro-2H-1,4-benzoxazine-8-carboxamide, (R)-6-chloro-4-methyl-N-(3-quinuclidinyl)-3,4-dihydro-2H-1,4-benzoxazine-8-carboxamide, (S)-6-chloro-4-methyl-N-(3-quinuclidinyl)-3,4-dihydro-2H-1,4-benzoxazine-8-carboxamide, 6-chloro-2,2,4-trimethyl-N-(3-quinuclidinyl)-3,4-dihydro-2H-1,4-benzoxazine-8-carboxamide, (R)-6-chloro-2,2,4-trimethyl-N-(3-quinuclidinyl)-3,4-dihydro-2H-1,4-benzoxazine-8-carboxamide, (S)-6-chloro-2,2,4-trimethyl-N-(3-quinuclidinyl)-3,4-dihydro-2H-1,4-benzoxazine-8-carboxamide, 6-fluoro-4-methyl-N-(3-quinuclidinyl)-3,4-dihydro-2H-1,4-benzoxazine-8-carboxamide, 6-chloro-N-(3-quinuclidinyl)-3,4-dihydro-2H-1,4-benzoxazine-8-carboxamide, 4-acetyl-6-chloro-N-(3-quinuclidinyl)-3,4-dihydro-2H-1,4-benzoxazine-8-carboxamide and 6-chloro-4-methyl-N-(8-methyl-8-azobicyclo[3.2.1]oct-3-yl)-3,4-dihydro-2H-1,4-benzoxazine-8-carboxamide and pharmaceutically acceptable salts thereof are preferred.

The compounds of formula (I) according to the present invention can be prepared by the following methods.

(1) Compounds of general formula (I) wherein m, q, and s are 0 can be produced by reacting a carboxylic acid represented by formula (II) as mentioned above or a reactive derivative thereof with a compound represented by formula (III)

$$R^9 - YH \qquad \text{(III)}$$

(wherein each symbol has the same meaning as that defined above).

(a) In the case where the compound of formula (II) is a free carboxylic acid, the reaction is carried out in an inert solvent, with cooling or heating or at room temperature, in the presence of a condensing agent such as dicyclohexylcarbodiimide, titanium tetrachloride, a phosphorus halide (phosphorus trichloride, phosphorus oxychloride, etc.), diethyl chlorophosphite, o-phenylene chlorophosphite, ethyl dichlorophosphite, or the like. Compound (III) may be treated beforehand with a phosphorus halide in an inert solvent before it is subjected to condensation with compound (II). For example, in the case where the phosphorus halide is phosphorus trichloride, compound (III) is treated beforehand, with cooling or at room temperature, with about 1/2 mol of phosphorus trichloride in an inert solvent in the presence of a tertiary base such as triethylamine, pyridine, N,N-dimethylaniline, or the like, and the resulting compound (III) is then reacted with compound (II) in an inert solvent at room temperature or with heating, preferably with heat refluxing.

(b) In the case where a reactive derivative of the carboxylic acid of formula (II) is employed and the derivative is an acid halide such as acid chloride or acid bromide, the reaction is carried out in an inert solvent, with cooling or at room temperature, in the presence of a tertiary base such as triethylamine, pyridine, N,N-dimethylaniline, or the like, or the reaction is carried out in water, with cooling or at room temperature, in the presence of an alkali such as sodium hydroxide, potassium hydroxide, or the like.

(c) In the case where as a reactive derivative of compound (II), use is made of a symmetric acid anhydride or a mixed anhydride such as an alkylcarbonic mixed anhydride, an alkylphosphate mixed anhydride, an alkylphosphite mixed anhydride, an alkylsulfuric mixed anhydride, or the like, the reaction is carried out in an inert solvent, with cooling or heating or at room temperature, in the presence of a tertiary base such as triethylamine, pyridine, N,N-dimethylaniline, N-methylmorpholine, or the like.

(d) In the case where as a reactive derivative of compound (II), an active amide such as an acid imidazolide, an acid pyrrolidide, 2,4-dimethylpyrazolide, or the like is used, the reaction is conducted in an inert solvent at room temperature or with heating.

(e) In the case where the compound of formula (III) is one in which Y is NH, this compound may also be reacted with that reactive derivative of compound (II) which is an ester such as a methyl ester, an ethyl ester, a p-nitrophenyl ester, a p-chlorophenyl ester, or the like. This reaction is effected in an inert solvent (the compound (III) may be used in excess so as to serve also as a solvent) at room temperature or with heating, preferably with heat refluxing.

The inert solvent used in each of the above-described condensation reactions can be benzene, toluene, xylene, methanol, ethanol, isopropyl alcohol, diethyl ether, dioxane, tetrahydrofuran, chloroform, dichloromethane, dichloroethane, hexamethylphosphoric triamide, diethylene glycol, dimethylformamide, ethyl acetate, or the like, or a mixture of these solvents. In the case where a reactive derivative of compound (II) is used, a proper solvent is selected according to the kind of the derivative.

The compound of general formula (II) can, for example, be produced by the following reaction routes.

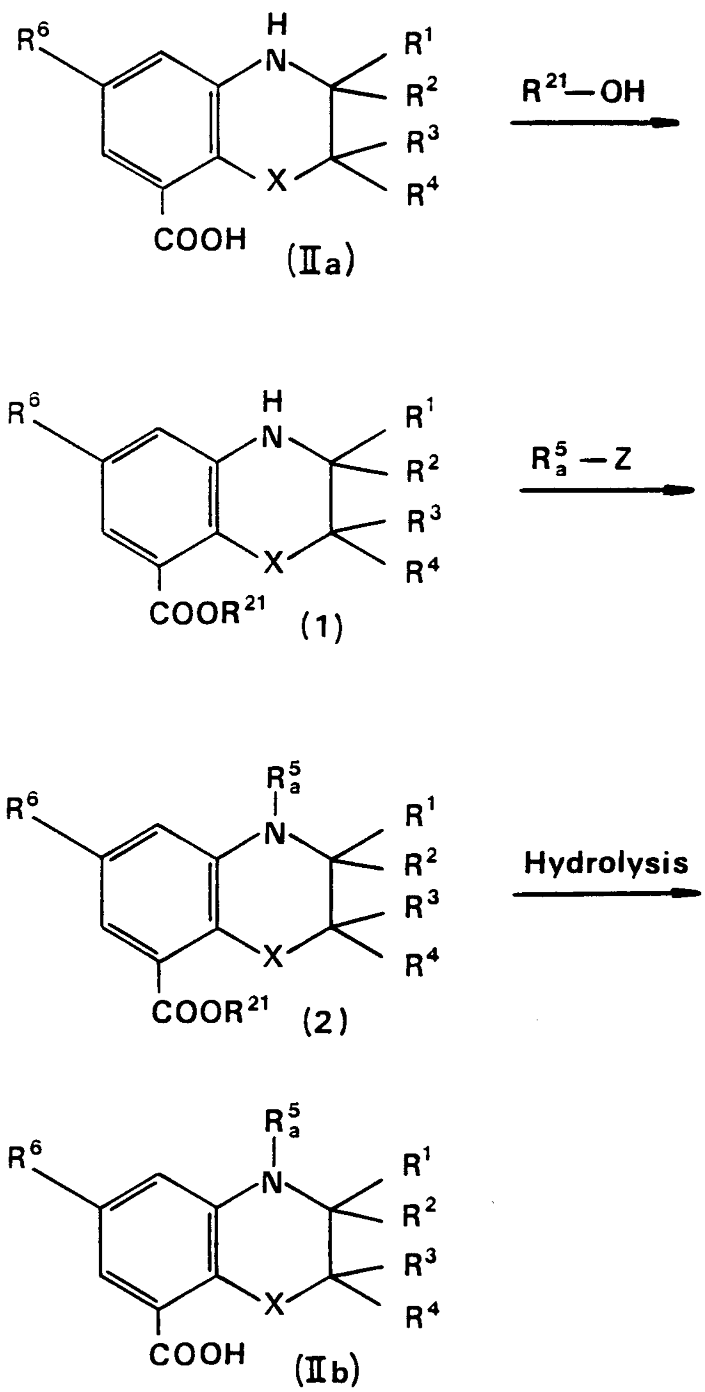


(IIa) (1) (2) (IIb)

(In the above route, $R^5_a$ represents the same group as $R^5$ excluding hydrogen, $R^{21}$ represents an ester residue, Z represents a group to be eliminated which may be chlorine, bromine, iodine, methanesulfonyloxy, p-toluenesulfonyloxy, or the like, and the other symbols have the same meanings as those defined above.)

Compound (IIa) for use in the above process can be obtained according to the method proposed by G. Coudert et al. (Synthesis, p.541, 1979). Compound (IIb) can be prepared by reacting compound (1), which is obtained by the esterification of compound (IIa), with $R^5_a$-Z in the presence of a tertiary base such as, for example, triethylamine or an inorganic salt such as, for example, potassium carbonate, and hydrolyzing the thus-obtained compound (2)

with an alkali.

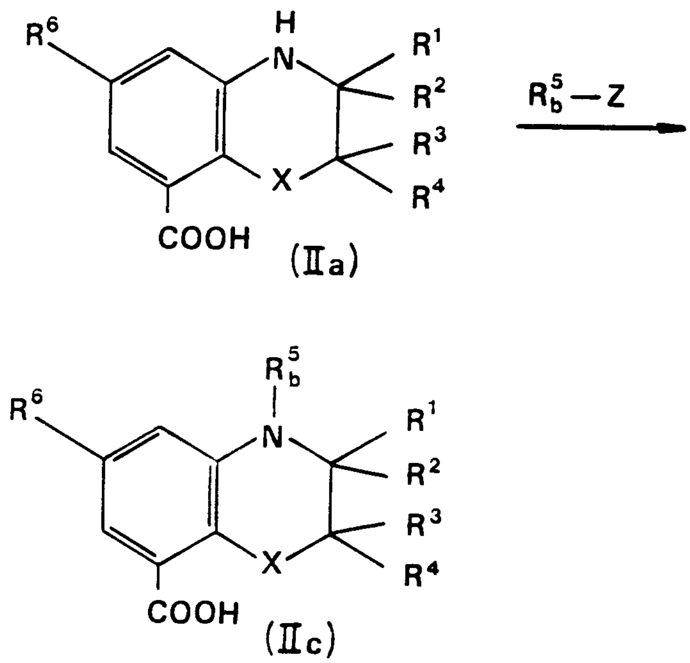


(IIa)

(IIc)

(In the above route, $R^5_b$ represents an alkanoyl, an aroyl, or a heteroaroyl and the other symbols have the same meanings as those defined above.)

Compound (IIc) can be produced by reacting compound (IIa) with $R^5_b$-Z in the presence of a tertiary base such as, for example, triethylamine or an inorganic salt such as, for example, potassium carbonate.

(2) Compounds of formula (I) wherein m, q, and s are 1 can be prepared by subjecting to an oxidation reaction those compounds of formula (I) wherein m, q, and s are 0 which are obtained by method (1).

The reaction is normally carried out in an inert solvent (such as chloroform, dichloromethane, tetrahydrofuran, dioxane, dimethylformamide, acetic acid or water, or a mixed solvent consisting of these) at a temperature ranging from -50°C to room temperature, preferably from -20°C to 0°C, for 5 minutes to 24 hours, preferably for 5 minutes to 6 hours. As an oxidizing agent such as metachloroperbenzoic acid, perbenzoic acid, peracetic acid, trifluoroperacetic acid, permaleic acid, sodium bromite, sodium hypochlorite or hydrogen peroxide, may be used. The oxidizing agent is normally used in an amount of from 1 to 10 equivalents, preferably from 1 equivalent to a slightly excess amount. This oxidation reaction may be performed in the presence of a catalyst such as sodium tungstate or the like.

Of the compounds of the present invention which are represented by formula (I) wherein $R^1$, $R^2$, $R^3$, and $R^4$ are different, those containing a chiral carbon are obtained in the form of a racemic mixture. The optical isomers in each of such racemic mixtures are within the scope of the present invention. If desired, such a racemic mixture can be optically divided by an ordinary method that employs an optically active acid (such as tartaric acid, dibenzoyltartaric acid, mandelic acid or 10-camphorsulfonic acid) to utilize the basicity of the racemic mixture. Further, an intended compound (I) having a desired configuration can be stereo selectively produced by subjecting to the above-described condensation reaction an optically active carboxylic acid obtained by optically dividing the racemate (II) by use of an optically active base (such as cinchonine, cinchonidine, brucine, quinine or $\alpha$-methylbenzylamine) or a reactive derivative of the optically active carboxylic acid together with an optically active compound (III) separately prepared by dividing with an optically active acid (such as tartaric acid, dibenzoyltartaric acid, mandelic acid or 10-camphorsulfonic acid).

Geometrical isomers also can be prepared according to ordinary methods.

The compound of general formula (I) can be converted into a pharmaceutically acceptable acid-adduct salt, such as hydrochloride, hydrobromide, phosphate, sulfate, p-toluenesulfonate, citrate, lactate, maleate, fumarate or tartrate

The following experiments will illustrate potent pharmacological activities of the compounds (I) of the present invention.

Pharmacological experiment 1: Antagonistic effect against von Bezold-Jarish reflex

5-$HT_3$ receptor blocking effects of the compounds of the present invention were evaluated based on the antagonistic effects against van Bezold-Jarish reflex caused by administering serotonin to anesthetized rats as an index according to Forzard's method described in Naunyuschmiedeberg's Arch. Pharmacol., vol.326, pp36, 1984.

Male Wistar rats weighing 350-450 g were anesthetized with an intraperitoneal injection of 1.25g/kg of urethane. The left jugular vein was cannulated for the intravenous injection and the left femoral vein was cannulated for the measurement of blood pressure and heart rate. Serotonin (20 µg/kg) was intravenously injected, and the compounds of the present invention were intravenously injected 5 minutes before the challenge with serotonin. The antagonistic activity of the compounds of the present invention against the caused reflex bradycardia was measured and the effective dose ($ED_{50}$, µg/kg), the dose required to antagonize the maximum activity by 50%, was determined. The results are summarized in Table 1.

Table 1

| Test Compound (Example No.) | $ED_{50}$ (µg/kg, i.v.) |
|---|---|
| 1 | 0.28 |
| 7 | 0.73 |
| 8 | 0.096 |
| 3 | 0.12 |
| 4 | 0.17 |
| 5 | 0.11 |

Pharmacological experiment 2: Dopamine $D_2$ binding test

Specific dopamine $D_2$ receptor binding test was conducted in accordance with the method described in European Journal of Pharmacology, vol.46, page 377, 1977.

Crude synaptosome fraction was separated from corpus striatum of 9 to 10 weeks-aged Wistar rats and suspended into 50 mM tris-hydrochloric acid buffer solution (pH 7.1) containing 120 mM sodium chloride, 5 mM potassium chloride, 2 mM calcium chloride, 1 mM magnesium chloride, 10 µM pargyline and 0.1% ascorbic acid.

Next, to the resulting synaptosome suspension were added the test compound of the present invention at several concentrations and tritium Spiperone at the terminal concentration of 0.2 nM, and the mixture was allowed to react at 37°C for 20 minutes. After completion of the reaction, the reaction mixture was suction filtered through Whatman GF/B glass filter. The glass filter was washed with 50 mM tris-hydrochloric acid buffer solution (pH 7.7), and then the radioactivity of the residue remaining on the glass filter was measured by liquid scintillation counter. Non-specific binding was determined under the presence of M(±)-Sulpiride. 50% Inhibition concentration ($IC_{50}$) of the test compound was graphically determined. The results are summarized in Table 2.

Pharmacological experiment 3: Serotonin binding test

Specific serotonin 1A (5-$HT_{1A}$) receptor binding test was conducted in accordance with the manner described in Journal of the Neurochemistry, vol.44, page 1685, 1985.

Crude synaptosome fraction was separated from hippocampus of 9 to 10 weeks-aged Wistar rat and suspended into 50 mM tris-hydrochloric acid buffer solution (pH 7.4) containing 1 mM manganese chloride.

Next, to the resulting synaptosome suspension were added the test compound of the present invention at several concentrations and tritium 8-OH-DPAT at the terminal concentration of 0.2 nM, and the mixture was allowed to react at 37°C for 12 minutes. After completion of the reaction, the reaction mixture was suction filtered through Whatman GF/B glass filter. The glass filter was washed with 50 mM tris-hydrochloric acid buffer solution (pH 7.4), and then the radioactivity of the residue remaining on the glass filter was measured by liquid scintillation counter. Non-specific binding was determined under the presence of $10^{-5}$M serotonin (5-HT). 50% Inhibition concentration ($IC_{50}$) of test compound was

graphically determined. The results are summarized in Table 2.

Table 2

| Test Compound (Example No.) | Dopamine $D_2$ binding test $IC_{50}$ (M) | Serotonin binding test $IC_{50}$ (M) |
|---|---|---|
| 13 | - | $7.5X10^{-7}$ |
| 14 | - | $6.2X10^{-8}$ |
| 38 | $6.9X10^{-7}$ | $2.4X10^{-8}$ |
| 39 | $6.3X10^{-7}$ | $4.2X10^{-7}$ |

All ddy male mice survived by the oral administration (300 mg/kg) and the intraperitoneal injection (100 mg/kg) of the test compounds of the present invention for 5 days.

As apparent from the results of various pharmacological experimentations including the experiments above, the compounds of the present invention show a 5-$HT_3$ receptor-blocking action and possess affinities with 5-$HT_{1A}$ and dopamine $D_2$ receptors, and are hence, useful for the prophylaxis or treatment of digestive diseases such as indigestion, delayed gastric emptying, diarrhea, indefinite complaint of alimentary system, irritable colon syndrome, peptic ulcer and/or the treatment for migraine headache, cluster headache, arrhythmia, or nausea and vomiting, especially, nausea and vomiting induced by the administration of carcinostatic substances, nausea and vomiting induced by radiotherapy, disturbances in central nervous systems such as dementia, depression, anxiety, psychopathy, drug abuse and material dependence, or the treatment for dystrophy or sentimental affection such as excitement or aggression accompanied by dyskinesia, senescence, cerebrovascular disease, or alcohol dependence.

In order that the compounds of the present invention be used as drugs and safely administered to patients, a therapeutically effective amount of the compounds are normally admixed with pharmaceutically acceptable carriers, excipients, diluents, and other ingredients and formulated into tablets (including sugar-coated tablets and film-coated tablets), granules, powders, injections, etc. The dosage may vary depending on the condition, body weight, age, etc. of the patient, but it may generally be about 0.1 to 100 mg/kg per day for an adult in the case of oral administration. The daily administration is preferably effected at a time or effected in several doses.

FORMULATION EXAMPLE 1

| | |
|---|---|
| Compound obtained in Example 1 | 10.0 mg |
| Lactose | 30.0 mg |
| Corn starch | 19.8 mg |
| Crystalline cellulose | 28.0 mg |
| Talc | 2.0 mg |
| Magnesium stearate | 0.2 mg |
| Total | 90.0 mg |

The compound obtained in Example 1, lactose, corn starch, and crystalline cellulose were mixed and kneaded, with a part of the corn starch being used as a binder paste, and the resulting blend was granulated and then dried at 50°C for 3 hours.

The dry granules were passed through a 24-mesh sieve, and then talc and magnesium stearate were added to the granules. The resulting mixture was formed into tablets each weighing 90 mg, by means of a rotary pelletizing machine of the punching type employing a pounder having a diameter of 6.0 mm. Subsequently, a film coating containing hydroxypropyl methyl cellulose and titanium oxide as base materials was formed over each of the above-obtained tablets in an amount of 5 mg per tablet.

FORMULATION EXAMPLE 2

| | |
|---|---|
| Compound obtained in Example 1 | 5.0 mg |
| Sodium chloride | 18.0 mg |
| Distilled water for injection in total | 2.0 ml |

Sodium chloride was dissolved in about 80 parts of water for use in injection, and the compound obtained in Example 1 was then added thereto and dissolved. Subsequently, the volume of the solution was adjusted to the total volume (100 parts). The resulting solution was filtered through a membrane filter (0.2 μm), charged into a 2-ml ampoule, and then sterilized at 115°C for 30 minutes, thereby producing an injection.

The present invention will be explained below in detail by means of the following Reference Examples and Examples, but the present invention should not be limited to these Examples.

REFERENCE EXAMPLE 1

A mixture of 12 g of 6-chloro-3,4-dihydro-2H-1,4-benzoxazine-8-carboxylic acid, 200 ml of methanol, and 10 ml of concentrated sulfuric acid was heat-refluxed for 24 hours with stirring. The liquid reaction mixture was cooled, and the crystals precipitated were filtered off, washed with methanol, and then dried, thereby obtaining 9.2 g of methyl 6-chloro-3,4-dihydro-2H-1,4-benzoxazine-8-carboxylate. Melting point: 60-61°C.

REFERENCE EXAMPLE 2

To a solution of 9.2 g of methyl 6-chloro-3,4-dihydro-2H-1,4-benzoxazine-8-carboxylate and 6.7 g of potassium carbonate in 100 ml of dimethylformamide was added 6.4 g of methyl iodide with cooling and stirring. The resulting mixture was heated at 70°C for 2 hours with stirring. The liquid reaction mixture was added to 200 ml of water, and the resulting insoluble substance was filtered off, washed with water, and dried, thereby obtaining 7.1 g of methyl 6-chloro-4-methyl-3,4-dihydro-2H-1,4-benzoxazine-8-carboxylate. Melting point: 91-92°C.

REFERENCE EXAMPLE 3

7.1 Grams of methyl 6-chloro-4-methyl-3,4-dihydro-2H-1,4-benzoxazine-8-carboxylate and 3.8 g of sodium hydroxide were dissolved in a mixed solvent obtained by mixing 50 ml of methanol and 100 ml of water, and the resulting solution was heated at 70°C for 2 hours with stirring. The liquid reaction mixture was added to 300 ml of water, and 6 ml of concentrated hydrochloric acid was added thereto. The resulting insoluble substance was filtered off, washed with water, and dried, thereby obtaining 5.8 g of 6-chloro-4-methyl-3,4-dihydro-2H-1,4-benzoxazine-8-carboxylic acid. Melting point: 202-204°C.

REFERENCE EXAMPLE 4

A solution of 10 g of 6-chloro-3,4-dihydro-2H-1,4-benzoxazine-8-carboxylic acid and 6.5 ml of triethylamine in 100 ml of chloroform was cooled, and 4.4 g of acetyl chloride was added thereto with stirring. The resulting mixture was stirred at room temperature for 2 hours. To the liquid reaction mixture were added an aqueous solution of sodium hydrogen carbonate and chloroform, and the resulting organic layer was separated, washed with water, and then dried with magnesium sulfate. The solvent was distilled off under reduced pressure, thereby obtaining 7.8 g of 4-acetyl-6-chloro-3,4-dihydro-2H-1,4-benzoxazine-8-carboxylic acid. Melting point: 76-77°C.

According to methods similar to Reference Examples above, following compounds were produced.

6-Chloro-4-ethyl-3,4-dihydro-2H-1,4-benzoxadine-8-carboxylic acid. Melting point: 76-77°C.

4-Butyl-6-chloro-3,4-dihydro-2H-1,4-benzoxazine-8-carboxylic acid. Melting point: 116-118°C.

6-Chloro-4-(2-phenylethyl)-3,4-dihydro-2H-1,4-benzoxazine-8-carboxylic acid. Melting point: 128-129°C.

6-Chloro-4-(2-chlorobenzyl)-3,4-dihydro-2H-1,4-benzoxazine-8-carboxylic acid. Melting point: 308°C (decomposed).

6-Chloro-4-(3-chlorobenzyl)-3,4-dihydro-2H-1,4-benzoxadine-8-carboxylic acid. Melting point: 292°C (decomposed).

6-Chloro-4-(4-chlorobenzyl)-3,4-dihydro-2H-1,4-benzoxazine-8-carboxylic acid. Melting point: 163-164°C.

4-Benzoyl-6-chloro-3,4-dihydro-2H-1,4-benzoxazine-8-carboxylic acid. Melting point: 175-176°C.

6-Chloro-4-(4-fluorobenzyl)-3,4-dihydro-2H-1,4-benzoxazine-8-carboxylic acid. Melting point: 133-135°C.
6-Fluoro-4-methyl-3,4-dihydro-2H-1,4-benzoxazine-8-carboxylic acid. Melting point: 164-166°C.
6-Bromo-4-methyl-3,4-dihydro-2H-1,4-benzoxazine-8-carboxylic acid. Melting point: 192-194°C.
4-Acetyl-3,4-dihydro-2H-1,4-benzoxazine-8-carboxylic acid. Melting point: 143°C.
4-Acetyl-6-nitro-3,4-dihydro-2H-1,4-benzoxazine-8-carboxylic acid. Melting point 243°C (decomposed).
4-Methyl-3,4-dihydro-2H-1,4-benzoxazine-8-carboxylic acid. Melting point: 117°C.
4-Methyl-6-nitro-3,4-dihydro-2H-1,4-benzoxazine-8-carboxylic acid. Melting point: 235°C (decomposed).
4-Methyl-6-sulfamoyl-3,4-dihydro-2H-1,4-benzoxazine-8-carboxylic acid. Melting point: 234°C (decomposed).
4-Methyl-6-methylthio-3,4-dihydro-2H-1,4-benzoxazine-8-carboxylic acid. Melting point: 170-172°C.
6-Methanesulfonyl-4-methyl-3,4-dihydro-2H-1,4-benzoxazine-8-carboxylic acid. Melting point: 215-217°C.

EXAMPLE 1

A solution of 2.1 g of 6-chloro-4-methyl-3,4-dihydro-2H-1,4-benzoxazine-8-carboxylic acid and 1.1 g of N-methylmorpholine in 50 ml of dimethylformamide was cooled to 5°C or below, and 1.5 g of isobutyl chlorocarbonate was added thereto with stirring. Stirring was then continued for 1 hour. To the liquid reaction mixture was added 1.6 g of 3-aminoquinuclidine, and the resulting mixture was stirred at room temperature for 5 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from ethanol-ethyl acetate, thereby obtaining 6-chloro-4-methyl-N-(3-quinuclidinyl)-3,4-dihydro-2H-1,4-benzoxazine-8-carboxamide hydrochloride. Melting point: 162°C (decomposed).

EXAMPLE 2

A solution of 3.75 g of 4-acetyl-6-chloro-3,4-dihydro-2H-1,4-benzoxazine-8-carboxylic acid and 1.5 g of N-methylmorpholine in 50 ml of dimethylformamide was cooled to 5°C or below, and 2.2 g of isobutyl chlorocarbonate was added thereto with stirring. Stirring was then continued for 1 hour. To the liquid reaction mixture was added 2.4 g of 3-aminoquinuclidine, and the resulting mixture was stirred at room temperature for 5 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from isopropyl alcohol, thereby obtaining 4-acetyl-6-chloro-N-(3-quinuclidinyl)-3,4-dihydro-2H-1,4-benzoxazine-8-carboxamide hydrochloride. Melting point: 155°C (decomposed).

EXAMPLE 3

1.53 Grams of 6-chloro-2,2,4-trimethyl-3,4-dihydro-2H-1,4-benzoxazine-8-carboxylic acid was dissolved in 30 ml of ethyl acetate, and 0.848 g of triethylamine was added thereto. While the resulting mixture was being cooled with ice at -10 to -5°C, 0.794 g of pivaloyl chloride was added dropwise. After this mixture was stirred at that temperature for 15 minutes, 0.906 g of 3-aminoquinuclidine was added at a temperature of -10 to -5°C, and the resulting mixture was stirred at room temperature for 1 hour. The liquid reaction mixture was washed with water, dried with anhydrous magnesium sulfate, and then condensed. Ethanolic hydrochloric acid was added to the residue, and the crystals precipitated were filtered off and then recrystallized from ethanol, thereby obtaining 6-chloro-2,2,4-trimethyl-N-(3-quinuclidinyl)-3,4-dihydro-2H-1,4-benzoxazine-8-carboxamide hydrochloride as colorless crystals. Melting point: 266°C (decomposed).

EXAMPLE 4

1.53 Grams of 6-chloro-2,2,4-trimethyl-3,4-dihydro-2H-1,4-benzoxazine-8-carboxylic acid was dissolved in 30 ml of ethyl acetate, and 0.848 g of triethylamine was added thereto. While the resulting mixture was being cooled with ice at -10 to -5°C, 0.794 g of pivaloyl chloride was added dropwise. After this mixture was stirred at that temperature for 15 minutes, 0.906 g of (R)-3-aminoquinuclidine was added at a temperature of -10 to -5°C, and the resulting mixture was stirred at room temperature for 1 hour. The liquid reaction mixture was washed with water, dried with anhydrous magnesium sulfate, and then condensed. Ethanolic hydrochloric acid was added to the residue, and the crystals precipitated were filtered off and then recrystallized from ethanol, thereby obtaining (R)-6-chloro-2,2,4-trimethyl-N-(3-quinuclidinyl)-3,4-dihydro-2H-1,4-benzoxazine-8-carboxamide hydrochloride as colorless crystals. Melting point: 272°C (decomposed). $[\alpha]_D$ = -3.6° (c = 0.5, $H_2O$).

EXAMPLE 5

1.53 Grams of 6-chloro-2,2,4-trimethyl-3,4-dihydro-2H-1,4-benzoxazine-8-carboxylic acid was dissolved in 30 ml of ethyl acetate, and 0.848 g of triethylamine was added thereto. While the resulting mixture was being cooled with ice at -10 to -5°C, 0.794 g of pivaloyl chloride was added dropwise. After this mixture was stirred at that temperature for 15 minutes, 0.906 g of (S)-3-aminoquinuclidine was added at a temperature of -10 to -5°C, and the resulting mixture was stirred at room temperature for 1 hour. The liquid reaction mixture was washed with water, dried with anhydrous magnesium sulfate, and then condensed. Ethanolic hydrochloric acid was added to the residue, and the crystals precipitated were filtered off and then recrystallized from ethanol, thereby obtaining (S)-6-chloro-2,2,4-trimethyl-N-(3-quinuclidinyl)-3,4-dihydro-2H-1,4-benzoxazine-8-carboxamide hydrochloride as colorless crystals. Melting point: 273°C (decomposed). $[\alpha]_D$ = +3.4° (c=0.5, $H_2O$).

EXAMPLE 6

A solution of 5 g of 6-chloro-3,4-dihydro-2H-1,4-benzoxazine-8-carboxylic acid and 2.8 g of N-methylmorpholine in 50 ml of dimethylformamide was cooled to 5°C or below, and 3.3 g of isobutyl chlorocarbonate was added thereto with stirring. Stirring was then continued for 30 minutes. To the liquid reaction mixture was added 3.7 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from ethanol-ethyl acetate, thereby obtaining 6-chloro-N-(3-quinuclidinyl)-3,4-dihydro-2H-1,4-benzoxazine-8-carboxamide hydrochloride. Melting point: 212-214°C.

EXAMPLE 7

A solution of 5 g of 6-chloro-4-methyl-3,4-dihydro-2H-1,4-benzoxazine-8-carboxylic acid and 2.8 g of N-methylmorpholine in 170 ml of tetrahydrofuran was cooled to 5°C or below, and 3.0 g of isobutyl chlorocarbonate was added thereto with stirring. Stirring was then continued for 30 minutes. To the liquid reaction mixture was added 2.8 g of (R)-3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from ethyl acetate, thereby obtaining (R)-6-chloro-4-methyl-N-(3-quinuclidinyl)-3,4-dihydro-2H-1,4-benzoxazine-8-carboxamide. Melting point: 186-187°C. $[\alpha]_D$ = +16.7° (c=1, Ethanol).

EXAMPLE 8

A solution of 5 g of 6-chloro-4-methyl-3,4-dihydro-2H-1,4-benzoxazine-8-carboxylic acid and 2.8 g of N-methylmorpholine in 170 ml of tetrahydrofuran was cooled to 5°C or below, and 3.0 g of isobutyl chlorocarbonate was added thereto with stirring. Stirring was then continued for 30 minutes. To the liquid reaction mixture was added 2.8 g of (S)-3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from ethyl acetate, thereby obtaining (S)-6-chloro-4-methyl-N-(3quinuclidinyl)-3,4-dihydro-2H-1,4-benzoxazine-8-carboxamide. Melting point: 186-187°C. $[\alpha]_D$ = -16.22° (c=1, Ethanol).

EXAMPLE 9

A solution of 5 g of 6-chloro-4-methyl-3,4-dihydro-2H-1,4-benzoxazine-8-carboxylic acid and 2.8 g of N-methylmorpholine in 170 ml of tetrahydrofuran was cooled to 5°C or below, and 3.0 g of isobutyl chlorocarbonate was added thereto with stirring. Stirring was then continued for 30 minutes. To the liquid reaction mixture was added 2.55 g of N,N-diethylaminoethylamine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from isopropyl alcohol, thereby obtaining 6-chloro-4-methyl-N-[2-(N,N-diethylamino)ethyl]-3,4-dihydro-2H-1,4-benzoxazine-8-carboxamide hydrochloride. Melting point: 195-197°C.

EXAMPLE 10

A solution of 3.5 g of 6-chloro-4-methyl-3,4-dihydro-2H-1,4-benzoxazine-8-carboxylic acid and 3.3 g of N-methylmorpholine in 100 ml of tetrahydrofuran was cooled to 5°C or below, and 2.3 g of isobutyl chlorocarbonate was added thereto with stirring. Stirring was then continued for 30 minutes. To the liquid reaction mixture was added 3 g of 4-amino-1-benzylpiperidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-methyl-N-(1-benzyl-4-piperidyl)-3,4-dihydro-2H-1,4-benzoxazine-8-carboxamide fumarate. Melting point: 232-233°C.

EXAMPLE 11

A solution of 2.3 g of 6-chloro-4-methyl-3,4-dihydro-2H-1,4-benzoxazine-8-carboxylic acid and 2.8 g of triethylamine in 30 ml of ethyl acetate was cooled to 5°C or below, and 1.3 g of pivaloyl chloride was added thereto with stirring. Stirring was then continued for 30 minutes. To the liquid reaction mixture was added 2.1 g of 3-amino-1-benzylpiperidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from isopropyl alcohol, thereby obtaining 6-chloro-4-methyl-N-(1-benzyl-3-piperidyl)-3,4-dihydro-2H-1,4-benzoxazine-8-carboxamide. Melting point: 109-110°C.

EXAMPLE 12

A solution of 1.30 g of 6-chloro-4-methyl-3,4-dihydro-2H-1,4-benzoxazine-8-carboxylic acid and 1.15 g of triethylamine in 20 ml of ethyl acetate was cooled to 5°C or below, and 0.79 g of pivaloyl chloride was added thereto with stirring. Stirring was then continued for 30 minutes. To the liquid reaction mixture was added 0.80 g of 8-methyl-8-azabicyclo[3.2.1]octan-3-amine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from isopropyl alcohol, thereby obtaining 6-chloro-4-methyl-N-(8-methyl-8-azabicyclo[3.2.1]oct-3-yl)-3,4-dihydro-2H-1,4-benzoxazine-8-carboxamide. Melting point: 183-185°C.

EXAMPLE 13

A solution of 4.5 g of 6-chloro-4-methyl-3,4-dihydro-2H-1,4-benzoxazine-8-carboxylic acid and 4.0 g of triethylamine in 60 ml of ethyl acetate was cooled to 5°C or below, and 2.7 g of pivaloyl chloride was added thereto with stirring. Stirring was then continued for 30 minutes. To the liquid reaction mixture was added 2.5 g of 2-aminomethyl-1-ethylpyrrolidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from ethanol, thereby obtaining 6-chloro-4-methyl-N-[(1-ethyl-2-pyrrolidinyl)methyl]-3,4-dihydro-2H-1,4-benzoxazine-8-carboxamide hydrochloride. Melting point: 197°C (decomposed).

EXAMPLE 14

A solution of 4.5 g of 6-chloro-4-methyl-3,4-dihydro-2H-1,4-benzoxazine-8-carboxylic acid and 4.0 g of triethylamine in 60 ml of ethyl acetate was cooled to 5°C or below, and 2.7 g of pivaloyl chloride was added thereto with stirring. Stirring was then continued for 30 minutes. To the liquid reaction mixture was added 3.1 g of 2-aminomethyl-1-butylpyrrolidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with ethanolic hydrochloric acid to convert the residue into a hydrochloride form. This hydrochloride was recrystallized from ethanol, thereby obtaining 6-chloro-4-methyl-N-[(1-butyl-2-pyrrolidinyl)-methyl]-3,4-dihydro-2H-1,4-benzoxazine-8-carboxamide hydrochloride. Melting point: 158-159°C.

EXAMPLE 15

A solution of 2.0 g of 4-benzyl-6-chloro-3,4-dihydro-2H-1,4-benzoxazine-8-carboxylic acid and 1.46 g of N-methylmorpholine in 100 ml of tetrahydrofuran was cooled to 5°C or below, and 0.99 g of isobutyl chlorocarbonate was added thereto with stirring. Stirring was then continued for 30 minutes. To the liquid reaction mixture was added 1.07 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 4-benzyl-6-chloro-N-(3-quinuclidinyl)-3,4-dihydro-2H-1,4-benzoxazine-8-carboxamide fumarate. Melting point: 237-239°C.

EXAMPLE 16

A solution of 2.0 g of 4-benzoyl-6-chloro-3,4-dihydro-2H-1,4-benzoxazine-8-carboxylic acid and 1.27 g of triethylamine in 60 ml of ethyl acetate was cooled to 5°C or below, and 0.86 g of pivaloyl chloride was added thereto with stirring. Stirring was then continued for 30 minutes. To the liquid reaction mixture was added 0.79 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of tartaric acid to convert the residue into a tartrate form. This tartrate was recrystallized from isopropyl alcohol, thereby obtaining 4-benzoyl-6-chloro-N-(3-quinuclidinyl)-3,4-dihydro-2H-1,4-benzoxazine-8-carboxamide tartrate. Melting point: 197°C (decomposed).

EXAMPLE 17

A solution of 1.4 g of 6-chloro-4-ethyl-3,4-dihydro-2H-1,4-benzoxazine-8-carboxylic acid and 1.17 g of triethylamine in 50 ml of ethyl acetate was cooled to 5°C or below, and 0.79 g of pivaloyl chloride was added thereto with stirring. Stirring was then continued for 30 minutes. To the liquid reaction mixture was added 0.73 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-ethyl-N-(3-quinuclidinyl)-3,4-dihydro-2H-1,4-benzoxazine-8-carboxamide fumarate. Melting point: 209-211°C.

EXAMPLE 18

A solution of 2.0 g of 6-chloro-4-butyl-3,4-dihydro-2H-1,4-benzoxazine-8-carboxylic acid and 1.50 g of triethylamine in 50 ml of ethyl acetate was cooled to 5°C or below, and 0.89 g of pivaloyl chloride was added thereto with stirring. Stirring was then continued for 30 minutes. To the liquid reaction mixture was added 0.93 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into fumarate form. This fumarate was recrystallized from ethanol, thereby obtaining 6-chloro-4-butyl-N-(3-quinuclidinyl,)-3,4-dihydro-2H-1,4-benzoxazine-8-carboxamide fumarate. Melting point: 193-194°C.

EXAMPLE 19

A solution of 2.0 g of 6-chloro-4-isobutyl-3,4-dihydro-2H-1,4-benzoxazine-8-carboxylic acid and 1.50 g of triethylamine in 50 ml of ethyl acetate was cooled to 5°C or below, and 0.89 g of pivaloyl chloride was added thereto with stirring. Stirring was then continued for 30 minutes. To the liquid reaction mixture was added 0.93 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-isobutyl-N-(3-quinuclidinyl)-3,4-dihydro-2H-1,4-benzoxazine-8-carboxamide fumarate. Melting point: 204-206°C.

EXAMPLE 20

A solution of 2.0 g of 6-chloro-4-(4-fluorobenzyl)-3,4-dihydro-2H-1,4-benzoxazine-8-carboxylic acid and 1.25 g of triethylamine in 50 ml of ethyl acetate was cooled to 5°C or below, and 0.75 g of pivaloyl chloride was added thereto with stirring. Stirring was then continued for 30 minutes. To the liquid reaction mixture was added 0.78 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-(4-fluorobenzyl)-N-(3-quinuclidinyl)-3,4-dihydro-2H-1,4-benzoxazine-8-carboxamide fumarate. Melting point: 191-192°C.

EXAMPLE 21

A solution of 2.0 g of 6-chloro-4-(4-methoxybenzyl)-3,4-dihydro-2H-1,4-benzoxazine-8-carboxylic acid and 1.24 g of triethylamine in 50 ml of ethyl acetate was cooled to 5°C or below, and 0.74 g of pivaloyl chloride was added thereto with stirring. Stirring was then continued for 30 minutes. To the liquid reaction mixture was added 0.77 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-(4-methoxybenzyl)-N-(3-quinuclidinyl)-3,4-dihydro-2H-1,4-benzoxazine-8-carboxamide fumarate. Melting point: 159-161°C.

EXAMPLE 22

A solution of 1.0 g of 6-chloro-4-(2-phenylethyl)-3,4-dihydro-2H-1,4-benzoxazine-8-carboxylic acid and 0.67 g of triethylamine in 40 ml of ethyl acetate was cooled to 5°C or below, and 0.40 g of pivaloyl chloride was added thereto with stirring. Stirring was then continued for 30 minutes. To the liquid reaction mixture was added 0.42 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-(2-phenylethyl)-N-(3-quinuclidinyl)-3,4-dihydro-2H-1,4-benzoxazine-8-carboxamide fumarate. Melting point: 185-186°C.

EXAMPLE 23

A solution of 1.5 g of 6-chloro-4-(4-chlorobenzyl)-3,4-dihydro-2H-1,4-benzoxazine-8-carboxylic acid and 0.94 g of triethylamine in 40 ml of ethyl acetate was cooled to 5°C or below, and 0.56 g of pivaloyl chloride was added thereto with stirring. Stirring was then continued for 30 minutes. To the liquid reaction mixture was added 0.59 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro--(4-chlorobenzyl)-N-(3-quinuclidinyl)-3,4-dihydro-2H-1,4-benzoxazine-8-carboxamide fumarate. Melting point: 223-225°C.

EXAMPLE 24

A solution of 2.0 g of 6-chloro-4-(2-chlorobenzyl)-3,4-dihydro-2H-1,4-benzoxazine-8-carboxylic acid and 1.25 g of triethylamine in 50 ml of ethyl acetate was cooled to 5°C or below, and 0.74 g of pivaloyl chloride was added thereto with stirring. Stirring was then continued for 30 minutes. To the liquid reaction mixture was added 0.78 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-(2-chlorobenzyl)-N-(3-quinuclidinyl)-3,4-dihydro-2H-1,4-benzoxazine-8-carboxamide fumarate. Melting point: 220-222°C.

EXAMPLE 25

A solution of 1.1 g of 6-fluoro-4-methyl-3,4-dihydro-2H-1,4-benzoxazine-8-carboxylic acid and 1.06 g of triethylamine in 40 ml of ethyl acetate was cooled to 5°C or below, and 0.63 g of pivaloyl chloride was added thereto with stirring. Stirring was then continued for 30 minutes. To the liquid reaction mixture was added 0.66 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-fluoro-4-methyl-N-(3-quinuclidinyl)-3,4-dihydro-2H-1,4-benzoxazine-8-carboxamide fumarate. Melting point: 201°C (decomposed).

EXAMPLE 26

A solution of 1.1 g of 6-bromo-4-methyl-3,4-dihydro-2H-1,4-benzoxazine-8-carboxylic acid and 1.06 g of triethylamine in 40 ml of ethyl acetate was cooled to 5°C or below, and 0.63 g of pivaloyl chloride was added thereto with stirring. Stirring was then continued for 30 minutes. To the liquid reaction mixture was added 0.66 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-bromo-4-methyl-N-(3-quinuclidinyl)-3,4-dihydro-2H-1,4-benzoxazine-8-carboxamide fumarate. Melting point: 198°C (decomposed).

EXAMPLE 27

A solution of 1.1 g of 4,6-dimethyl-3,4-dihydro-2H-1,4-benzoxazine-8-carboxylic acid and 1.06 g of triethylamine in 40 ml of ethyl acetate was cooled to 5°C or below, and 0.63 g of pivaloyl chloride was added thereto with stirring. Stirring was then continued for 30 minutes. To the liquid reaction mixture was added 0.66 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 4,6-dimethyl-N-(3-quinuclidinyl)-3,4-dihydro-2H-1,4-benzoxazine-8-carboxamide fumarate. Melting point: 234°C (decomposed).

EXAMPLE 28

A solution of 1.5 g of 6-chloro-4-methyl-3,4-dihydro-2H-1,4-benzoxazine-8-carboxylic acid and 1.34 g of triethylamine in 40 ml of ethyl acetate was cooled to 5°C or below, and 0.79 g of pivaloyl chloride was added thereto with stirring. Stirring was then continued for 30 minutes. To the liquid reaction mixture was added 0.86 g of 1-(2-aminoethyl)morpholine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol-hydrochloric acid to convert the residue into a hydrochloride form. This hydrochloride was recrystallized from ethanol, thereby obtaining 6-chloro-4-methyl-N-(2-morpholinoethyl)-3,4-dihydro-2H-1,4-benzoxazine-8-carboxamide hydrochloride. Melting point: 190-191°C.

EXAMPLE 29

A solution of 1.5 g of 6-chloro-4-methyl-3,4-dihydro-2H-1,4-benzoxazine-8-carboxylic acid and 1.34 g of triethylamine in 40 ml of ethyl acetate was cooled to 5°C or below, and 0.79 g of pivaloyl chloride was added thereto with stirring. Stirring was then continued for 30 minutes. To the liquid reaction mixture was added 0.85 g of 1-(2-aminoethyl)piperidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol-hydrochloric acid to convert the residue into a hydrochloride form. This hydrochloride was recrystallized from ethanol, thereby obtaining 6-chloro-4-methyl-N-(2-piperidinoethyl)-3,4-dihydro-2H-1,4-benzoxazine-8-carboxamide hydrochloride. Melting point: 137-139°C.

EXAMPLE 30

A solution of 1.5 g of 6-chloro-4-methyl-3,4-dihydro-2H-1,4-benzoxazine-8-carboxylic acid and 1.34 g of triethylamine in 40 ml of ethyl acetate was cooled to 5°C or below, and 0.79 g of pivaloyl chloride was added thereto with stirring. Stirring was then continued for 30 minutes. To the liquid reaction mixture was added 0.77 g of 1-(2-aminoethyl)pyrrolidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, thereby obtaining 6-chloro-4-methyl-N-[2-(1-pyrrolidinyl)ethyl]-3,4-dihydro-2H-1,4-benzoxazine-8-carboxamide. Melting point: 88-89°C.

EXAMPLE 31

A solution of 1.5 g of 6-chloro-4-methyl-3,4-dihydro-2H-1,4-benzoxazine-8-carboxylic acid and 1.34 g of triethylamine in 40 ml of ethyl acetate was cooled to 5°C or below, and 0.79 g of pivaloyl chloride was added thereto with stirring. Stirring was then continued for 30 minutes. To the liquid reaction mixture was added 0.91 g of 3-aminomethyl-1-benzylmorpholine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanolhydrochloric acid to convert the residue into a hydrochloride form. This hydrochloride was recrystallized from ethanol, thereby obtaining 6-chloro-4-methyl-N-[1-benzyl-3-morpholinyl)methyl]-3,4-dihydro-2H-1,4-benzoxazine-8-carboxamide hydrochloride. Melting point: 154-156°C.

EXAMPLE 32

To a solution of 3.0 g of 6-chloro-4-methyl-3,4-dihydro-2H-1,4-benzoxazine-8-carboxylic acid in 50 ml of dichloroethane was added 1.7 g of thionyl chloride and stirred for 1 hour. The reaction mixture was concentrated to obtain 3.1 of crude 6-chloro-4-methyl-3,4-dihydro-2H-1,4-benzoxazine-8-carboxylic acid chloride.

Next, a solution of 1.4 g of 3-hydroxyquinuclidine in 30 ml of tetrahydrofuran was cooled to 0°C or below, and 5 ml of hexane solution of 15% n-butyllithium was added thereto with stirring. Stirring was then continued for 30 minutes. To this solution was then added 3.1 g of 6-chloro-4-methyl-3,4-dihydro-2H-1,4-benzoxazine-8-carboxylic acid chloride as obtained above and stirred for 1 hour. To this mixture was then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 6-chloro-4-ethyl-N-(3-quinuclidinyl)-3,4-dihydro-2H-1,4-benzoxazine-8-carboxylate fumarate. Melting point: 167-168°C.

EXAMPLE 33

A solution of 2.0 g of 4-acetyl-3,4-dihydro-2H-1,4-benzoxazine-8-carboxylic acid and 2.5 g of triethylamine in 50 ml of ethyl acetate was cooled to 5°C or below, and 1.2 g of pivaloyl chloride was added thereto with stirring. Stirring was then continued for 30 minutes. To the liquid reaction mixture was added 1.26 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 4-acetyl-N-(3-quinuclidinyl)-3,4-dihydro-2H-1,4-benzoxazine-8-carboxamide fumarate. Melting point: 196-197°C.

EXAMPLE 34

A solution of 5.0 g of 4-acetyl-6-nitro 3,4-dihydro-2H-1,4-benzoxazine-8-carboxylic acid and 5.6 g of triethylamine in 80 ml of ethyl acetate was cooled to 5°C or below, and 2.5 g of pivaloyl chloride was added thereto with stirring. Stirring was then continued for 30 minutes. To the liquid reaction mixture was added 2.65 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from isopropyl ether, thereby obtaining 4-acetyl-6-nitro-N-(3-quinuclidinyl)-3,4-dihydro-2H-1,4-benzoxazine-8-carboxamide. Melting point: 280°C (decomposed).

EXAMPLE 35

A solution of 2.0 g of 4-methyl-3,4-dihydro-2H-1,4-benzoxazine-8-carboxylic acid and 2.0 g of triethylamine in 50 ml of ethyl acetate was cooled to 5°C or below, and 1.46 g of pivaloyl chloride was added thereto with stirring. Stirring was then continued for 30 minutes. To the liquid reaction mixture was added 1.53 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was treated with an ethanol solution of fumaric acid to convert the residue into a fumarate form. This fumarate was recrystallized from ethanol, thereby obtaining 4-methyl-N-(3-quinuclidinyl)-3,4-dihydro-2H-1,4-benzoxazine-8-carboxamide fumarate. Melting point: 178-180°C.

EXAMPLE 36

A solution of 4.0 g of 4-methyl-6-nitro-3,4-dihydro-2H-1,4-benzoxazine-8-carboxylic acid and 5.1 g of triethylamine in 50 ml of ethyl acetate was cooled to 5°C or below, and 2.3 g of pivaloyl chloride was added thereto with stirring. Stirring was then continued for 30 minutes. To the liquid reaction mixture was added 2.4 g of 3-aminoquinuclidine, and the resulting mixture was stirred at room temperature for 2 hours. To this mixture were then added an aqueous solution of sodium hydrogen carbonate and ethyl acetate, and the resulting organic layer was separated, washed with water, and dried with magnesium sulfate. The solvent was distilled off under reduced pressure, and the residue was recrystallized from isopropyl ether, thereby obtaining 4-methyl-6-nitro-N-(3-quinuclidinyl)-3,4-dihydro-2H-1,4-benzoxazine-8-carboxamide. Melting point: 224-226°C.

EXAMPLE 37

To a solution of 1.8 g of 4-methyl-6-nitro-N-(3-quinuclidinyl)-3,4-dihydro-2H-1,4-benzoxazine-8-carboxamide in 50 ml of methanol was added 1.0 g of Raney nickel to conduct reduction reaction under atmospheric pressure with charging hydrogen gas. The catalyst was filtered out and the solvent was distilled off under reduced pressure to thereby obtaining 6-amino-4-methyl-N-(3-quinuclidinyl)-3,4-dihydro-2H-1,4-benzoxazine-8-carboxamide.

NMR spectra ($CDCl_3$; ppm): $\delta$=2.88 (s, 3H), 3.32 (t, 2H), 4.32 (t, 2H), 6.13 (d, 1H), 6.82 (d, 1H), 7.9-8.2 (br, 1H).

EXAMPLE 38

A solution of 2.0 g of 6-chloro-4-methyl-3,4-dihydro-2H-1,4-benzoxazine-8-carboxylic acid and 1.1 g of N-methylmorpholine in 10 ml of dimethylformamide and 20 ml of tetrahydrofuran was cooled to -15 to -20°C, and 1.2 g of a isobutyl chloroformate was added thereto with stirring. Stirring was then continued for 25 minutes. To the liquid reaction mixture was added 2.0 g of (R)-(+)-2-aminomethyl-1-nonylpyrrolidine, and the resulting mixture was stirred at room temperature for 2 hours. The resulting mixture was concentrated under reduced pressure to obtain a residue. To the residue were then added an aqueous solution of sodium hydrogen carbonate and ethyl acetate, and the resulting organic layer was separated, washed with water, and dried with magnesium sulfate. The solvent was distilled off under reduced pressure, and the residue was separated and purified by means of a silica gel column chromatography, thereby obtaining (R)-(+)-6-chloro-4-methyl-N-[(1-nonyl-2-pyrrolidinyl)methyl]-3,4-dihydro-2H-1,4-benzoxazine-8-carboxamide as oil. $[\alpha]_D$ = +58.3° (c=1, methanol).

EXAMPLE 39

A solution of 2.28 g of 6-chloro-4-methyl-3,4-dihydro-2H-1,4-benzoxazine-8-carboxylic acid and 3 ml of triethylamine in 10 ml of dimethylformamide and 50 ml of tetrahydrofuran was cooled to -15 to -20°C, and 1.45 ml of isobutylformamide was added thereto with stirring. Stirring was then continued for 20 minutes. To the liquid reaction mixture was added 1.7 g of (R)-(+)-2-aminomethyl-1-butylpyrrolidine, and the resulting mixture was stirred at room temperature for 4 hours. The reaction mixture was concentrated under reduced pressure to obtain a residue. To the residue were then added an aqueous solution of sodium hydrogen carbonate and ethyl acetate, and the resulting organic layer was separated, washed with water, and dried with magnesium sulfate. The solvent was distilled off under reduced pressure, and the residue was separated and purified by means of silica gel column chromatography, thereby obtaining (R)-(+)-N-[(1-butyl-2-pyrrolidinyl)methyl]-6-chloro-4-methyl-3,4-dihydro-2H-1,4-benzoxazine-8-carboxamide as oil. $[\alpha]_D$ = +62.7° (c=1, methanol).

EXAMPLE 40

A solution of 6.0 g of 4-methyl-6-nitro-3,4-dihydro-2H-1,4-benzoxazine-8-carboxylic acid and 5 g of N-methylmorpholine in 30 ml of dimethylformamide and 20 ml of tetrahydrofuran was cooled to -15 to -20°C, and 3.4 g of isobutyl chloroformate was added thereto with stirring. Stirring was then continued for 25 minutes. To the liquid reaction mixture was added 3.9 g of 2-amino-methyl-1-butylpyrrolidine, and the resulting mixture was stirred at room temperature for 4 hours. The reaction mixture was concentrated under reduced pressure to obtain a residue. To the residue were then added an aqueous solution of potassium carbonate and ethyl acetate, and the resulting organic layer was separated, washed with water, and dried with magnesium sulfate. The solvent was distilled off under reduced pressure, and the residue was recrystallized from isopropyl ether, thereby obtaining N-[(1-butyl-2-pyrrolidinyl)methyl]-4-methyl-6-nitro-3,4-dihydro-2H-1,4-benzoxazine-8-carboxamide. Melting point: 88-89°C.

EXAMPLE 41

A solution of 3 g of 4-methyl-3,4-dihydro-2H-1,4-benzoxazine-8-carboxylic acid and 2.4 g of N-methylmorpholine in 7 ml of dimethylformamide and 15 ml of tetrahydrofuran was cooled to -15 to -20°C, and 2.1 g of isobutylchlroformate was added thereto with stirring. Stirring was then continued for 20 minutes. To the liquid reaction mixture was added 2.4 g of 2-amino-methyl-1-butyl pyrrolidine, and the resulting mixture was stirred at room temperature for 4 hours. The reaction mixture was concentrated under reduced pressure to obtain a residue. To the residue were then added an aqueous solution of potassium carbonate and ethyl acetate, and the resulting organic layer was separated, washed with water, and dried with magnesium sulfate. The solvent was distilled off under reduced pressure, thereby obtaining N-[(1-butyl-2-pyrrolidinyl)methyl]-4-methyl-3,4-dihydro-2H-1,4-benzoxazine-8-carboxamide.
NMR spectra ($CDCl_3$; ppm): δ=0.9 (t, 3H), 2.9 (s, 3H), 3.3 (t, 2H), 4.38 (t, 2H), 6.6-7.6 (m, 3H), 7.9-8.3 (br, 1H).

EXAMPLE 42

A solution of 2 g of 4-methyl-6-sulfamoyl-3,4-dihydro-2H-1,4-benzoxazine-8-carboxylic acid and 1.1 g of N-methylmorpholine in 30 ml of dimethylformamide and 10 ml of tetrahydrofuran was cooled to -15 to -20°C, and 1 g of isobutyl chloroformate was added thereto with stirring. Stirring was then continued for 20 minutes. To the liquid reaction mixture was added 1.15 g of 2-amino-methyl-1-butylpyrrolidine, and the resulting mixture was stirred at room temperature for 4 hours. The reaction mixture was concentrated under reduced pressure to obtain a residue. To the residue were then added an aqueous solution of potassium carbonate and ethyl acetate, and the resulting organic layer was separated, washed with water, and dried with magnesium sulfate. The solvent was distilled off under reduced pressure, and the residue was recrystallized from isopropyl ether, thereby obtaining N-[(1-butyl-2-pyrrolidinyl)methyl]-4-methyl-6-sulfamoyl-3,4-dihydro-2H-1,4-benzoxazine-8- carboxamide. Melting point: 144-145°C.

EXAMPLE 43

A solution of 0.8 g of 4-methyl-6-methylthio-3,4-dihydro-2H-1,4-benzoxazine-8-carboxylic acid and 0.5 g of N-methylmorpholine in 5 ml of dimethylformamide and 5 ml of tetrahydrofuran was cooled to -15 to -20°C, and 0.45 g of isobutyl chloroformate was added thereto with stirring. Stirring was then continued for 20 minutes. To the liquid reaction mixture was added 0.52 g of 2-amino-methyl-1-butylpyrrolidine, and the resulting mixture was stirred at room temperature for 4 hours. To this mixture were then added an aqueous solution of potassium carbonate and ethyl acetate, and the resulting organic layer was separated, washed with water, and dried with magnesium sulfate. The solvent was distilled off under reduced pressure, thereby obtaining N-[(1-butyl-2-pyrrolidinyl)methyl]-4-methyl-6-methylthio-3,4-dihydro-2H-1,4-benzoxazine-8-carboxamide.
NMR spectra ($CDCl_3$; ppm): δ=0.9 (t, 3H), 2.46 (s, 3H), 2.9 (s, 3H), 3.3 (t, 2H), 4.36 (t, 2H), 6.67 (d, 1H), 7.42 (d, 1H), 7.9-8.3 (br, 1H).

EXAMPLE 44

To a solution of 2 g of N-[(1-butyl-2-pyrrolidinyl)methyl]-4-methyl-6-nitro-3,4-dihydro-2H-1,4-benzoxazine-8-carboxamide in 150 ml of methanol was added 0.7 g of palladium-on-carbon to conduct reduction reaction under atmospheric pressure with charging hydrogen gas. The catalyst was filtered out, and the solvent was distilled off under reduced pressure. The residue was treated with an ethanol solution of fumaric acid to convert the residue into fumarate form. This fumarate was recrystallized from ethanol, thereby obtaining 6-amino-N-[(1-butyl-2-pyrrolidinyl)methyl]-4-methyl-3,4-dihydro-2H-1,4-benzoxazine-8-carboxamide 2 fumarate. Melting point: 160-161°C (decomposed).

EXAMPLE 45

To a solution of 0.3 g of 6-chloro-2,2-diphenyl-4-methyl-2,3-dihydro-1,4-benzoxazine-8-carboxylic acid in 10 ml of ethyl acetate was added 0.16 g of triethylamine. To the mixture was added dropwise 0.098 g of pivaloyl chloride with ice-cooling to -10 to -5°C, and stirred for 15 minutes at the temperature. To the reaction mixture was added 0.103 g of 3-aminoquinuclidine at -10 to -5°C, and the resulting mixture was stirred at room temperature for 1 hour. The reaction mixture was then washed with water, dried with anhydrous magnesium sulfate, and concentrated. To the residue was added ethanolic hydrochloric acid, and the precipitated crystals were collected by filtration and recrystallized from ethanol, thereby obtaining 6-chloro-2,2-diphenyl-4-methyl-N-(3-quinuclidinyl)-2,3-dihydro-1,4-benzoxazine-8-carboxamide hydrochloride as colorless crystals. Melting point: 192-194°C.

EXAMPLE 46

A solution of 2 g of 6-chloro-4-methyl-3,4-dihydro-2H-1,4-benzoxazine-8-carboxylic acid and 1.3 g of N-methylmorpholine in 10 ml of dimethylformamide and 20 ml of tetrahydrofuran was cooled to -15 to -20°C, and 1.2 g of isobutyl chloroformate was added thereto with stirring. Stirring was then continued for 20 minutes. To the liquid reaction mixture was added 1.4 g of (s)-(-)-2-aminomethyl-1-butylpyrrolidine, and the resulting mixture was stirred at room temperature for 4 hours. The reaction mixture was concentrated under reduced pressure to obtain residue. To the residue were then added an aqueous solution of potassium carbonate and ethyl acetate, and the resulting organic layer was separated, washed with water, and dried with magnesium sulfate. The solvent was distilled off under reduced pressure, and the residue was separated and purified by means of silica gel column chromatography, thereby containing (s)-(-)-N[(1-butyl-2-pyrrolidinyl)methyl]-6-chloro-4-methyl-3,4-dihydro-2H-1,4-benzoxazine-8-carboxamide as oil. $[\alpha]_D$ = - 63.7°C (c=1, methanol).

EXAMPLE 47

A solution of 250 mg of 4-methyl-6-methylsulfonyl-3,4-dihydro-2H-1,4-benzoxazine-8-carboxylic acid and 186 mg of N-methylmorpholine in 4 ml of dimethylformamide and 4 ml of tetrahydrofuran was cooled to -15 to -20°C, and 126 mg of isobutyl chloroformate was added thereto with stirring. Stirring was then continued for 20 minutes. To the liquid reaction mixture was added 144 mg of 2-aminomethyl-1-butylpyrrolidine, and the resulting mixture was stirred at room temperature for 4 hours. To this mixture were then added an aqueous solution of potassium carbonate and ethyl acetate, and the resulting organic layer was separated, washed with water, and dried with magnesium sulfate. The solvent was distilled off under reduced pressure, and the residue was recrystallized from ethanol, thereby obtaining N-[(1-butyl-2-pyrrolidinyl)methyl]-4-methyl-6-methylsulfonyl-3,4-dihydro-2H-1,4-benzoxazine-8-carboxamide. Melting point: 110-112°C.

EXAMPLE 48

To a solution of 1.8 g of 6-chloro-4-methyl-2,3-dihydro-1,4-benzothiazine-8-carboxylic acid in 30 ml of ethyl acetate was added 1.05 g of triethylamine, and then added 0.891 g of pivaloyl chloride dropwise with stirring at -10°C to -5°C followed by stirring the mixture at the same temperature for 30 minutes. To the resultant mixture was added a solution of 1.12 g of 3-aminoquinuclidine in 15 ml of ethyl acetate at -10 to -5°C and then stirred at room temperature for an hour. After the reaction solution was washed with water, dried over anhydrous sodium sulfate and concentrated, the crystalline residue thus obtained was recrystallized from ethyl acetate to give 6-chloro-4-methyl-N-(3-quinuclidinyl)-2,3-dihydro-1,4-benzothiazine-8-carboxamide as yellow crystals. Melting point: 195 - 196°C.

EXAMPLE 49

To a solution of 1.8 g of 6-chloro-4-methyl-2,3-dihydro-1,4-benzothiazine-8-carboxylic acid in 30 ml of ethyl acetate was added 1.05 g of triethylamine, and then added 0.891 g of pivaloyl chloride dropwise with stirring at -10°C to -5°C followed by stirring the mixture at the same temperature for 30 minutes. To the resultant mixture was added 1.39 g of 2-aminomethyl-1-butylpyrrolidine at -10°C to -5°C and then stirred at room temperature for an hour. After the reaction solution was washed with water, dried over anhydrous sodium sulfate and concentrated, the residue thus obtained was crystallized from diisopropyl ether. The crystals were collected by filtration and recrystallized from diisopropyl ether to give N[(1-butyl-2-pyrrolidinyl)methyl]-6-chloro-4-methyl-2,3-dihydro-1,4-benzothiazine-8-carboxamide as colorless crystals. Melting point: 108-109°C.

According to methods similar to the above, the following compounds were produced.

6-Chloro-2,3,4-trimethyl-N-(3-quinuclidinyl)-3,4-dihydro-2H-1,4-benzoxadine-8-carboxamide.

6-chloro-2,4-dimethyl-N-(3-quinuclidinyl)-3,4-dihydro-2H-1,4-benzoxadine-8-carboxamide.

6-Chloro-3,4-dimethyl-N-(3-quinuclidinyl)-3,4-dihydro-2H-1,4-benzoxadine-8-carboxamide.
6-Chloro-2,2,3,3,4-pentamethyl-N-(3-quinuclidinyl)-3,4-dihydro-2H-1,4-benzoxadine-8-carboxamide.
6-Chloro-3,3,4-trimethyl-N-(3-quinuclidinyl)-3,4-dihydro-2H-1,4-benzoxadine-8-carboxamide.
6-Chloro-4-methyl-N-(3-quinuclidinyl)-4H-1,4-benzoxadine-8-carboxamide.
6-Chloro-3,4-dimethyl-N-(3-quinuclidinyl)-4H-1,4-benzoxadine-8-carboxamide.
6-Chloro-2,4-dimethyl-N-(3-quinuclidinyl)-4H-1,4-benzoxadine-8-carboxamide.
6-Chloro-N-(3-quinuclidinyl)-2H-1,4-benzoxadine-8-carboxamide.
6-Chloro-4-methyl-2,3-diphenyl-N-(3-quinuclidinyl)-3,4-dihydro-2H-1,4-benzoxadine-8-carboxamide.
6-Chloro-2,3,4-trimethyl-N-(3-quinuclidinyl)-3,4-dihydro-2H-1,4-benzothiazine-8-carboxamide.
6-Chloro-N-(3-quinuclidinyl)-2,3-dimethyl-3,4-dihydro-2H-1,4-benzoxadine-8-carboxamide.
6-Chloro-4-(N,N-diethylcarbamoyl)-N-(3-quinuclidinyl)-3,4-dihydro-2H-1,4-benzoxadine-8-carboxamide.
6-Chloro-4-(2-diethylaminoethyl)-N-(3-quinuclidinyl)-3,4-dihydro-2H-1,4-benzoxadine-8-carboxamide.
6-Hydroxy-4-methyl-N-(3-quinuclidinyl)-3,4-dihydro-2H-1,4-benzoxadine-8-carboxamide.
4-Methyl-6-methoxy-N-(3-quinuclidinyl)-3,4-dihydro-2H-1,4-benzoxadine-8-carboxamide.
3-Quinuclidinyl 6-chloro-4-methyl-3,4-dihydro-2H-1,4-benzoxadine-8-carboxylate.
3-Quinuclidinyl 6-chloro-2,3,4-trimethyl-3,4-dihydro-2H-1,4-benzoxadine-8-carboxylate.
6-Chloro-2,3,4-trimethyl-N-(8-methyl-8-azabicyclo[3.2.1]oct-3-yl)-3,4-dihydro-2H-1,4-benzoxadine-8-carboxamide.
6-Chloro-4-methyl-N-(9-methyl-9-azabicyclo[3.3.1]non-3-yl)-3,4-dihydro-2H-1,4-benzoxadine-8-carboxamide.
6-Chloro-2,3,4-trimethyl-N-(9-methyl-9-azabicyclo[3.3.1]non-3-yl)-3,4-dihydro-2H-1,4-benzoxadine-8-carboxamide.
6-Chloro-2,3,4-trimethyl-N-(1-benzyl-4-piperidyl)-3,4-dihydro-2H-1,4-benzoxadine-8-carboxamide.
6-Chloro-2,3,4-trimethyl-N-[2-(N,N-diethylamino)ethyl]-3,4-dihydro-2H-1,4-benzoxadine-8-carboxamide.
6-Chloro-4-methyl-N-(2-amino-4-thiazolylmethyl)-3,4-dihydro-2H-1,4-benzoxadine-8-carboxamide.
6-Chloro-2,3,4-trimethyl-N-(2-amino-4-thiazolyl-methyl)-3,4-dihydro-2H-1,4-benzoxadine-8-carboxamide.
N-[(1-Butyl-2-pyrrolidinyl)methyl]-6-methanesulfonyl-4-methyl-3,4-dihydro-2H-1,4-benzoxazine-8-carboxamide.

The present invention has been fully explained in the description and examples given above, but variations and modifications thereof may be made without departing from the scope of the present invention.

## Claims

### Claims for the following Contracting States : DE, GB, FR, IT, NL, SE, CH, LI, BE, AT, LU, DK

1. A benzazine compound, a geometrical isomer of said benzazine compound, an optical isomer of said benzazine compound, and a pharmaceutically acceptable salt of said benzazine compound, said benzazine compound being represented by formula (I):

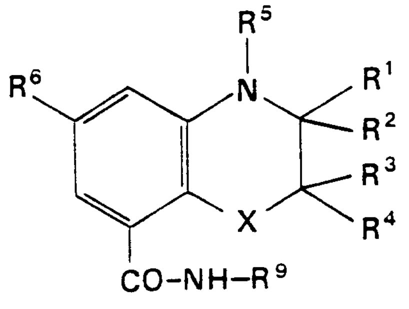

(I)

(wherein $R^1$, $R^2$, $R^3$, and $R^4$ are the same or different and each represents hydrogen; an alkyl having 1 to 20 carbon atoms, phenyl; a phenyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a phenylalkyl which is benzyl, 1- or 2-phenylethyl, 3-phenylpropyl or 4-phenylbutyl, a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a heteroaryl which is pyridyl, thienyl, furyl, imidazolyl, pyrazolyl or pyrimidinyl; a substituted heteroaryl which is pyridyl, thienyl, furyl, imidazolyl, pyrazolyl or pyrimidinyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon

atoms, carboxy, and alkoxycarbonyls in which the alkoxy moiety has 1 to 8 carbon atoms; a heteroarylalkyl in which the heteroaryl means pyridiyl, thienyl, furyl, imidazolyl, pyrazolyl or pyrimidinyl and the alkyl moiety has 1 to 8 carbon atoms; or a substituted heteroarylalkyl in which the heteroaryl means pyridyl, thienoyl, furyl, imidazolyl, pyrazolyl or pyrimidinyl and the alkyl moiety has 1 to 8 carbon atoms substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; or one of $R^1$ and $R^2$ is bonded to one of $R^3$ and $R^4$ to form a single bond; $R^5$ represents hydrogen; an alkyl having 1 to 20 carbon atoms; an alkanoyl having 2 to 5 carbon atoms; an aroyl which is benzoyl, naphthoyl, phenylacetyl, phenylpropionyl or phenylbutyryl; a substituted aroyl which is benzoyl, naphthoyl, phenylacetyl, phenylpropionyl or phenylbutyryl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy and alkoxycarbonyls in which the alkoxy moiety has 1 to 8 carbon atoms; a heteroaroyl which is 2- or 3-furoyl, 2- or 3-thienoyl, nicotinoyl or isonicotinoyl; a substituted heteroaroyl which is 2- or 3-furoyl, 2- or 3- thienoyl, nicotinoyl or isonicotinoyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; carbamoyl, a carbamoyl substituted by 1 or 2 alkyls having 1 to 8 carbon atoms, an aminoalkyl in which the alkyl moiety has 1 to 8 carbon atoms; or a mono- or di-substituted aminoalkyl in which the amino moiety has been substituted by methyl and the alkyl moiety has 1 to 8 carbon atoms; or $R^5$ may be bonded to one or $R^1$ and $R^2$ to form a single bond; $R^6$ represents hydrogen; a halogen; an alkyl having 1 to 20 carbon atoms; an alkoxy having 1 to 8 carbon atoms; amino; an acylamino which is alkanoylamino having 2 to 5 carbon atoms; a mono- or dialkyl-substituted amino in which the alkyl moiety has 1 to 8 carbon atoms; hydroxyl group; a protected hydroxyl group, nitro, a haloalkyl in which the alkyl moiety has 1 to 4 carbon atoms; cyano; $-S(O)_\ell$ $R^{18}$ ($R^{18}$ represents an alkyl having 1 to 20 carbon atoms, phenyl or a phenyl substituted by 1 to 3 substituents selected from halogens, alkoxyls having 1 to 8 carbon atoms; alkyls having 1 to 20 carbon atoms; nitro, amino, haloalkyl in which the alkyl moiety has 1 to 4 carbon atoms; carboxyl, or alkoxycarbonyls in which the alkoxy moiety has 1 to 8 carbon atoms and $\ell$ is 0, 1 or 2)
or

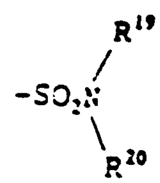

($R^{19}$ and $R^{20}$ are the same or different and each represents hydrogen or an alkyl having 1 to 20 carbon atoms); X represents oxygen or sulfur; and $R^9$ represents the formula:

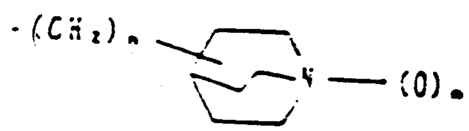

(m is 0 or 1 and n is 0, 1, or 2),

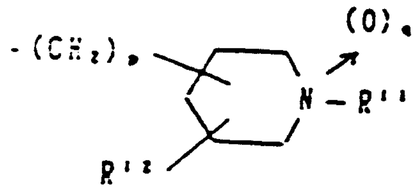

(p is 0, 1 or 2, q is 0 or 1, $R^{11}$ represents an alkyl having 1 to 20 carbon atoms; a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy and alkoxycarbonyls in which the alkoxy moiety has 1 to 8 carbon atoms; a phenoxyalkyl which is phenoxymethyl, 2-phenoxyethyl, 3-phenoxypropyl or 4-phenoxybutyl; or a substituted phenoxyalkyl which is phenoxymethyl, 2-phenoxyethyl, 3-phenoxypropyl or 4-phenoxybutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; and $R^{12}$ represents hydrdogen or an alkoxy having 1 to 8 carbon atoms),

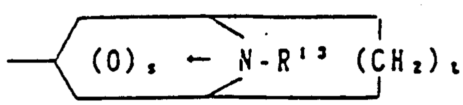

(s and t each is 0 or 1, and $R^{13}$ represents an alkyl having 1 to 20 carbon atoms; a phenylalkyl which is benzyl, 1- or 2-phenylethyl, 3-phenylpropyl or 4-phenylbutyl; or a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy and alkoxycarbonyls in which the alkoxy moiety has 1 to 8 carbon atoms),

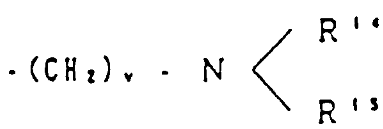

($R^{14}$ and $R^{15}$ are the same or different and each represents hydrogen; an alkyl having 1 to 20 carbon atoms; a phenylalkyl which is benzyl, 1- or 2-phenylethyl, 3-phenylpropyl or 4-phenylbutyl; or a substituted phenylalkyl which is benzyl, 1- or 2-phenylethyl, 3-phenylpropyl or 4-phenylbutyl, substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkokycarbonyls in which the alkoxy moiety has 1 to 8 carbon atoms; or $R^{14}$ and $R^{15}$ are bonded to each other to be a group which forms a heterocyclic ring in cooperation with the adjacent nitrogen atom, and v is an integer of 1 to 8), or

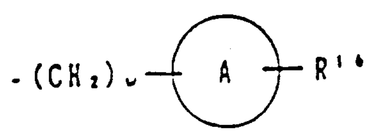

(ring A represents a condensed or uncondensed, nitrogen-containing heterocyclic ring group which may further

contain oxygen, sulfur or N-$R^{17}$ ($R^{17}$ represents hydrogen or an alkyl having 1 to 20 carbon atoms) in the ring thereof, $R^{16}$ represents hydrogen, an alkyl having 1 to 20 carbon atoms; an alkenyl having 2 to 20 carbon atoms; an alkynyl having 2 to 20 carbon atoms, a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens , alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; amino, a mono- or dialkyl-substituted amino in which the alkyl moiety has 1 to 8 carbon atoms; or an acylamino which is alkanoylamino having 2 to 5 carbon atoms, and w is 1, 2, 3 or 4)).

**2.** The compound of claim 1 wherein said compound is selected from the group consisting of 6-chloro-4-methyl-N-(3-quinuclidinyl)-3,4-dihydro-2H-1,4-benzoxazine- 8-carboxamide, (R)-6-chloro-4-methyl-N-(3-quinuclidinyl)-3,4-dihydro-2H-1,4-benzoxazine-8-carboxamide, (S)-6-chloro-4-methyl-N-(3-quinuclidinyl)-3,4-dihydro-2H-1,4-benzoxazine-8-carboxamide, 6-chloro-2,2,4-trimethyl-N-(3-quinuclidinyl)-3,4-dihydro-2H-1,4-benzoxazine-8-carboxamide, (R)-6-chloro-2,2,4-trimethyl-N-(3-quinuclidinyl)-3,4-dihydro-2H-1,4-benzoxazine-8-carboxamide, (S)-6-chloro-2,2,4-trimethyl-N-(3-quinuclidinyl)-3,4-dihydro-2H-1,4-benzoxazine-8-carboxamide, 6-fluoro-4-methyl-N-(3-quinuclidinyl)-3,4-dihydro-2H-1,4-benzoxazine-8-carboxamide, 6-chloro-N-(3-quinuclidinyl)-3,4-dihydro-2H-1,4-benzoxazine-8-carboxamide, 4-acetyl-6-chloro-N-(3-quinuclidinyl)-3,4-dihydro-2H-1,4-benzoxazine-8-carboxamide, 6-chloro-4-methyl-N-(8-methyl-8-azobicyclo-[3.2.1]oct-3-yl)-3,4-dihydro-2H-1,4-benzoxazine-8-carboxamide and pharmaceutically acceptable salts thereof.

**3.** A benzazine compound, a geometrical isomer of said benzazine compound, an optical isomer of said benzazine compound, and a pharmaceutically acceptable salt of said benzazine compound, said benzazine compound being represented by formula (II):

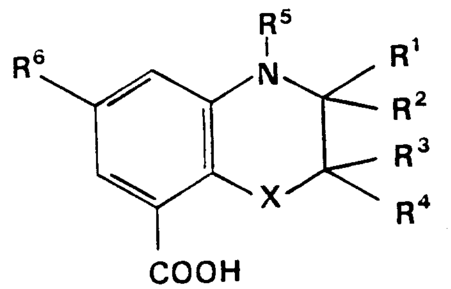

(II)

wherein each symbol has the same meaning as that defined in Claim 1 or a reactive derivative thereof.

**4.** A pharmaceutical composition comprising a compound as claimed in any one of the preceding claims and a pharmaceutically acceptable carrier, excipient or diluent.

**5.** The use of a compound as claimed in any one of claims 1 to 3 in the preparation of an pharmaceutical formulation for the treatment of a condition which requires an antagonist for 5-$HT_3$ receptors.

**Claims for the following Contracting State : ES**

**1.** A process for the preparation of a benzazine compound, a geometrical isomer of said benzazine compound, an optical isomer of said benzazine compound, and a pharmaceutically acceptable salt of said benzazine compound,

said benzazine compound being represented by formula (I):

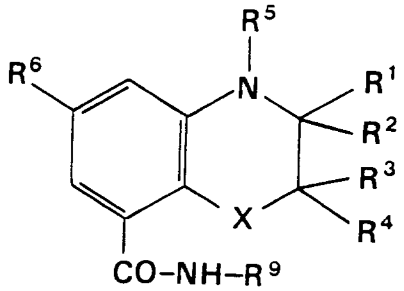

(I)

(wherein $R^1$, $R^2$, $R^3$, and $R^4$ are the same or different and each represents hydrogen, an alkyl having 1 to 20 carbon atoms, phenyl, a phenyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms, a phenylalkyl which is benzyl, 1- or 2-phenylethyl, 3-phenylpropyl or 4-phenylbutyl, a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a heteroaryl which is pyridyl, thienyl, furyl, imidazolyl, pyrazolyl or pyrimidinyl; a substituted heteroaryl which is pyridyl, thienyl, furyl, imidazolyl, pyrazolyl or pyrimidinyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a heteroarylalkyl in which the heteroaryl means pyridiyl, thienyl, furyl, imidazolyl, pyrazolyl or pyrimidinyl and the alkyl moiety has 1 to 8 carbon atoms; or a substituted heteroarylalxyl in which the heteroaryl means pyridyl, thienoyl, furyl, imidazolyl, pyrazolyl or pyrimidinyl and the alkyl moiety has 1 to 8 carbon atoms substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; or one of $R^1$ and $R^2$ is bonded to one of $R^3$ and $R^4$ to form a single bond; $R^5$ represents hydrogen; an alkyl having 1 to 20 carbon atoms; an alkanoyl having 2 to 5 carbon atoms; an aroyl which is benzoyl, naphthoyl, phenylacetyl, phenylpropionyl or phenylbutyryl; a substituted aroyl which is benzoyl, naphthoyl, phenylacetyl, phenylpropionyl or phenylbutyryl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy and alkoxycarbonyls in which the alkoxy moiety has 1 to 8 carbon atoms, a heteroaroyl which is 2- or 3-furoyl, 2- or 3-thienoyl, nicotinoyl or isonicotinoyl, a substituted heteroaroyl which is 2- or 3-furoyl, 2- or 3- thienoyl, nicotinoyl or isonicotinoyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; carbamoyl; a carbamoyl substituted by 1 or 2 alkyls having 1 to 8 carbon atoms; an aminoalkyl in which the alkyl moiety has 1 to 8 carbon atoms; or a mono- or di-substituted aminoalkyl in which the amino moiety has been substituted by methyl and the alkyl moiety has 1 to 8 carbon atoms; or $R^5$ may be bonded to one or $R^1$ and $R^2$ to form a single bond; $R^6$ represents hydrogen, a halogen; an alkyl having 1 to 20 carbon atoms; an alkoxy having 1 to 8 carbon atoms, amino; an acylamino which is alkanoylamino having 2 to 5 carbon atoms; a mono- or dialkyl-substituted amino in which the alkyl moiety has 1 to 8 carbon atoms, hydroxyl group; a protected hydroxyl group; nitro; a haloalkyl in which the alkyl moiety has 1 to 4 carbon atoms, cyano, $-S(O)_{\ell}R^{18}$ ($R^{18}$ represents an alkyl having 1 to 20 carbon atoms, phenyl or a phenyl substituted by 1 to 3 substituents selected from halogens, alkoxyls having 1 to 8 carbon atoms; alkyls having 1 to 20 carbon atoms; nitro, amino, haloalkyl in which the alkyl moiety has 1 to 4 carbon atoms; carboxyl, or alkoxy carbonyls in which the alkoxy moiety has 1 to 8 carbon atoms; and $\ell$ is 0, 1 or 2)

or

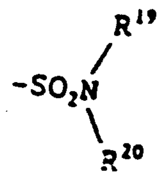

($R^{19}$ and $R^{20}$ are the same or different and each represents hydrogen or an alkyl having 1 to 20 carbon atoms); X represents oxygen or sulfur; and $R^9$ represents the formula:

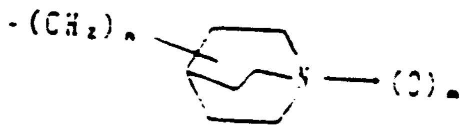

(m is 0 or 1 and n is 0, 1, or 2),

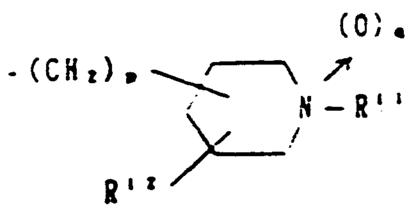

(p is 0, 1 or 2, q is 0 or 1, $R^{11}$ represents an alkyl having 1 to 20 carbon atoms; a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy and alkoxycarbonyls in which the alkoxy moiety has 1 to 8 carbon atoms; a phenoxyalkyl which is phenoxymethyl, 2-phenoxyethyl, 3-phenoxypropyl or 4-phenoxybutyl; or a substituted phenoxyalkyl which is phenoxymethyl, 2-phenoxyethyl, 3-phenoxypropyl or 4-phenoxybutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; and $R^{12}$ represents hydrdogen or an alkoxy having 1 to 8 carbon atoms),

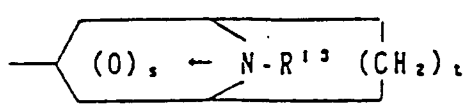

(s and t each is 0 or 1 and $R^{13}$ represents an alkyl having 1 to 20 carbon atoms; a phenylalkyl which is benzyl, 1- or 2-phenylethyl, 3-phenylpropyl or 4-phenylbutyl; or a substituted phenylalkyl which is benzyl, 1- or 2-phenylethyl, 3-phenylpropyl or 4-phenylbutyl substituted by 1 to 3 substituents selected from halogens, alkoxyls having 1 to 8 carbon atoms, alkyls having 1 to 20 carbon atoms, nitro, amino, haloalkyl in which the alkyl moiety has 1 to 4 carbon

atoms, carboxy and alkoxycarbonyls in which the alkoxy moiety has 1 to 8 carbon atoms),

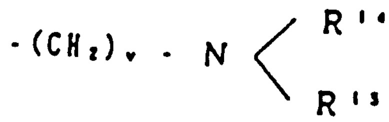

($R^{14}$ and $R^{15}$ are the same or different and each represents hydrogen; an alkyl having 1 to 20 carbon atoms; a phenylalkyl which is benzyl, 1- or 2-phenylethyl, 3-phenylpropyl or 4-phenylbutyl; or a substituted phenylalkyl which is benzyl, 1- or 2-phenylethyl, 3-phenylpropyl or 4-phenylbutyl, substituted by 1 to 3 substituents selected from halogens, alkoxyls having 1 to 8 carbon atoms, alkyls having 1 to 20 carbon atoms; nitro, amino, haloalkyl in which the alkyl moiety has 1 to 4 carbon atoms, carboxy, and alkokycarbonyls in which the alkoxy moiety has 1 to 8 carbon atoms; or $R^{14}$ and $R^{15}$ are bonded to each other to be a group which forms a heterocyclic ring in cooperation with the adjacent nitrogen atom, and v is an integer of 1 to 8), or

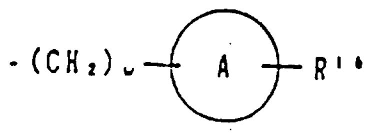

(ring A represents a condensed or uncondensed, nitrogen-containing heterocyclic ring group which may further contain oxygen, sulfur or N-$R^{17}$ ($R^{17}$ represents hydrogen or an alkyl having 1 to 20 carbon atoms) in the ring thereof, $R^{16}$ represents hydrogen, an alkyl having 1 to 20 carbon atoms; an alkenyl having 2 to 20 carbon atoms, an alkynyl having 2 to 20 carbon atoms; a phenylalkyl which is benzyl, 1- or 2-phenylethyl, 3-phenylpropyl or 4-phenylbutyl; a substituted phenylalkyl which is benzyl, 1- or 2-phenylethyl, 3-phenylpropyl or 4-phenylbutyl substituted by 1 to 3 substituents selected from halogens , alkoxyls having 1 to 8 carbon atoms, alkyls having 1 to 20 carbon atoms, nitro, amino, haloalkyl in which the alkyl moiety has 1 to 4 carbon atoms, carboxy, and alkoxycarbonyls in which the alkoxy moiety has 1 to 8 carbon atoms; amino, a mono- or dialkyl-substituted amino in which the alkyl moiety has 1 to 8 carbon atoms; or an acylamino which is alkanoylamino having 2 to 5 carbon atoms; and w is 1, 2, 3 or 4)), which process comprises reacting a benzazine compound, a geometrical isomer of said benzazine compound, an optical isomer of said benzazine compound, and a pharmaceutically acceptable salt of said benzazine compound, said benzazine compound being represented by formula (II)

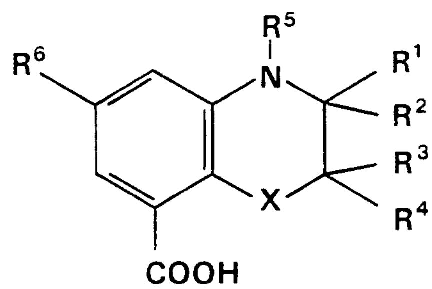

(II)

wherein each symbol has the same meaning as that defined above or a reactive derivative thereof with a compound represented by formula (III)

$$R^9 - NH_2$$

(wherein $R^9$ has the same meaning as that defined above).

**2.** A process as claimed in claim 1 wherein said compound is selected from the group consisting of 6-chloro-4-methyl-N-(3-quinuclidinyl)-3,4-dihydro-2H-1, 4-benzoxazine--8-carboxamide, (R)-6-chloro-4-methyl-N-(3-quinuclidinyl)-3,4-dihydro-2H-1,4-benzoxazine-8-carboxamide, (S)-6-chloro-4-methyl-N-(3-quinuclidinyl)-3,4-dihydro-2H-1,4-benzoxazine-8-carboxamide, 6-chloro-2,2,4-trimethyl-N-(3-quinuclidinyl)-3,4-dihydro-2H-1,4-benzoxazine-8-carboxamide, (R)-6-chloro-2,2,4-trimethyl-N-(3-quinuclidinyl)-3,4-dihydro-2H-1,4-benzoxazine-8-carboxamide, (S)-6-chloro-2,2,4-trimethyl-N-(3-quinuclidinyl)-3,4-dihydro-2H-1,4-benzoxazine-8-carboxamide, 6-fluoro-4-methyl-N-(3-quinuclidinyl)-3,4-dihydro-2H-1,4-benzoxazine-8-carboxamide, 6-chloro-N-(3-quinuclidinyl)-3,4-dihydro-2H-1,4-benzoxazine-8-carboxamide, 4-acetyl-6-chloro-N-(3-quinuclidinyl)-3,4-dihydro-2H-1,4-benzoxazine-8-carboxamide, 6-chloro-4-methyl-N-(8-methyl-8-azobicyclo-[3.2.1]oct-3-yl)-3,4-dihydro-2H-1,4-benzoxazine-8-carboxamide and pharmaceutically acceptable salts thereof.

3. A process for the preparation of a benzazine compound, a geometrical isomer of said benzazine compound, an optical isomer of said benzazine compound, and a pharmaceutically acceptable salt of said benzazine compound, said benzazine compound being represented by formula (II):

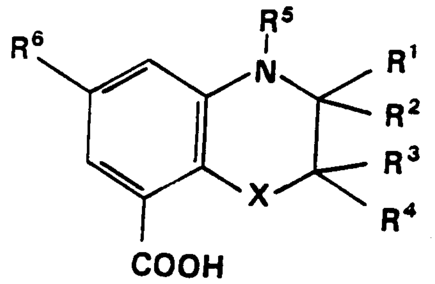

(II)

wherein each symbol has the same meaning as that defined in claim 1, or a reactive derivative thereof wherein said

compound is produced using the reaction route set out below:

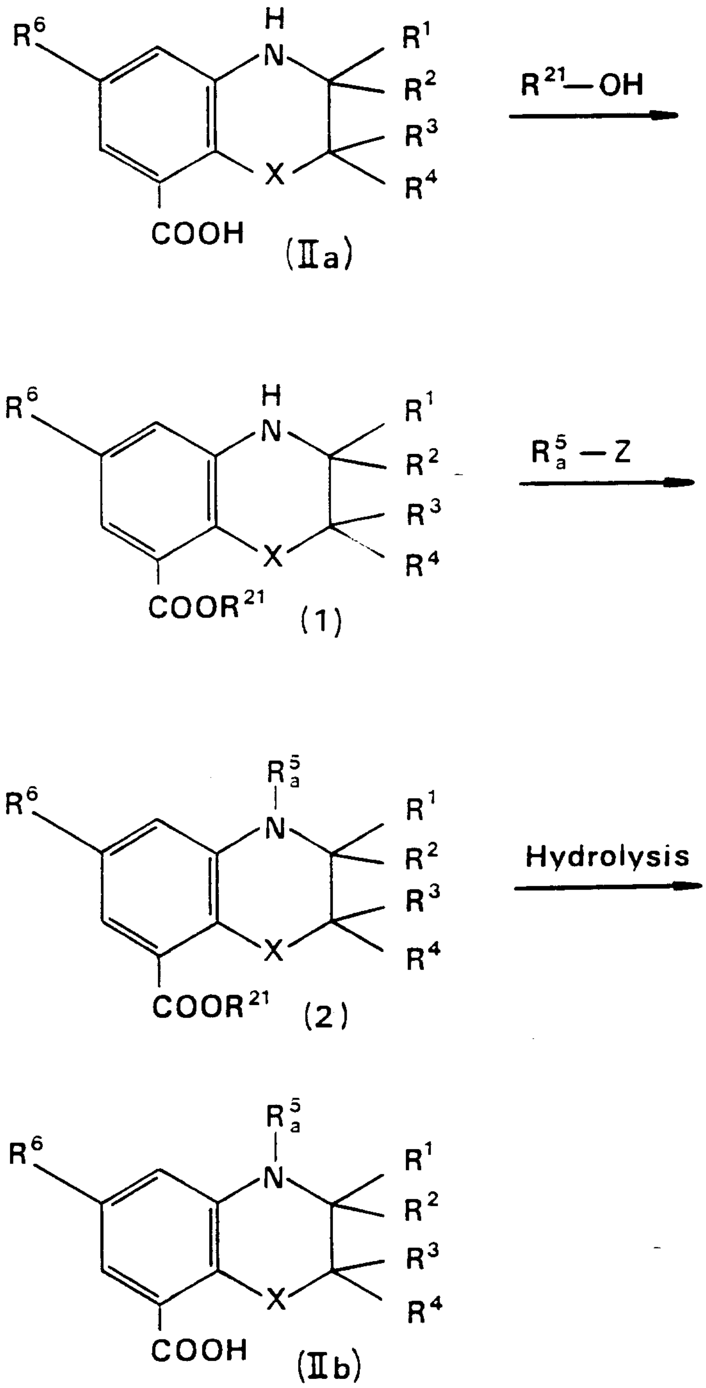


(IIa)

(1)

(2)

(IIb)

4. A process for the preparation of a benzazine compound as defined in Claim 1,which process comprises reacting a compound represented by formula (II):

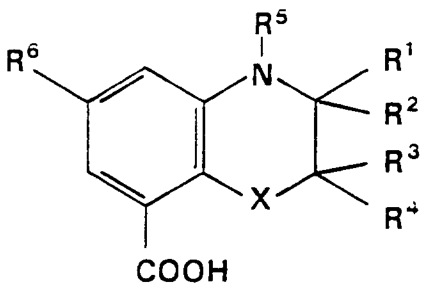

(II)

to provide the said benzazine compound.

## Patentansprüche

**Patentansprüche für folgende Vertragsstaaten : DE, GB, FR, IT, NL, SE, CH, LI, BE, AT, LU, DK**

1. Benzazinverbindung, ein geometrisches Isomer der Benzazinverbindung, ein optisches Isomer der Benzazinverbindung und ein pharmazeutisch verträgliches Salz der Benzazinverbindung, wobei die Benzazinverbindung durch die Formel (I) dargestellt wird:

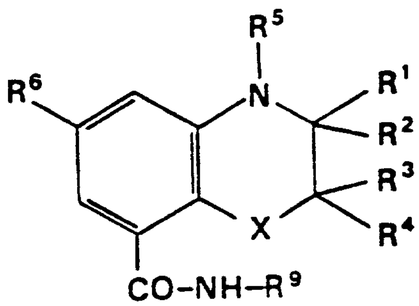

(I)

(worin $R^1$, $R^2$, $R^3$ und $R^4$ gleich oder verschieden sind, und jeweils Wasserstoff; ein Alkyl mit 1 bis 20 Kohlenstoffatomen; Phenyl; ein Phenyl, das mit 1 bis 3 Substituenten substituiert ist, die ausgewählt sind aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonyle, in welchen der Alkoxyrest 1 bis 8 Kohlenstoffatome aufweist;ein Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist, ein Heteroaryl; das Pyridyl, Thienyl, Furyl, Imidazolyl, Pyrazolyl oder Pyrimidinyl ist; ein substituiertes Heteroaryl, das Pyridyl, Thienyl, Furyl, Imidazolyl, Pyrazolyl oder Pyrimidinyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Heteroarylalkyl, worin Heteroaryl Pyridyl, Thienyl, Furyl, Imidazolyl, Pyrazolyl oder Pyrimidinyl bedeutet und der Alkylrest 1 bis 8 Kohlenstoffatome aufweist; oder ein substituiertes Heteroarylalkyl, worin Heteroaryl Pyridyl, Thienyl, Furyl, Imidazolyl, Pyrazolyl oder Pyrimidinyl bedeutet, worin der Alykylrest 1 bis 8 Kohlenstoffatome aufweist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist, bedeutet; oder worin einer von $R^1$ und $R^2$ an einen von $R^3$ und $R^4$ gebunden ist, um eine Einfachbindung

zu bilden, $R^5$ Wasserstoff, ein Alkyl mit 1 bis 20 Kohlenstoffatomen; ein Alkanoyl mit 2 bis 5 Kohlenstoffatomen; ein Aroyl das Benzoyl, Naphthoyl, Phenylacetyl, Phenylpropionyl oder Phenylbutyril ist; ein substituiertes Aroyl, das Benzoyl, Naphthoyl, Phenylacetyl, Phenylpropionyl oder Phenylbutyril ist, welches durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist;
ein Heteroaroyl, das 2- oder 3-Furoyl, 2- oder 3-Thienoyl, Nikotinoyl oder iso-Nikotinoyl ist; ein substituiertes Heteroaroyl, das 2- oder 3-Furoyl, 2- oder 3-Thienoyl, Nikotinoyl oder iso-Nikotinoyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Phenylalkyl das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; Carbamoyl, ein Carbamoyl, das durch 1 bis 2 Alkyle mit 1 bis 8 Kohlenstoffatomen substituiert ist; ein Aminoalkyl, worin der Alkylrest 1 bis 8 Kohlenstoffatome aufweist; oder ein mono- oder disubstituiertes Aminoalkyl, worin der Aminorest durch Methyl substituiert worden ist und der Alkylrest 1 bis 8 Kohlenstoffatome aufweist, bedeutet, oder worin $R^5$ an einen von $R^1$ oder $R^2$ gebunden sein kann, um eine Einfachbindung zu bilden;
$R^6$ Wasserstoff; ein Halogen, ein Alkyl mit 1 bis 20 Kohlenstoffatomen; ein Alkoxy mit 1 bis 8 Kohlenstoffatomen; Amino; ein Acylamino, das Alkanoylamino mit 2 bis 5 Kohlenstoffatomen ist; ein mono- oder di-Alkyl substituiertes Amino, worin der Alkylrest 1 bis 8 Kohlenstoffatome aufweist; eine Hydroxylgruppe; eine geschützte Hydroxylgruppe; Nitro; ein Haloalkyl, worin der Alkylrest 1 bis 4 Kohlenstoffatome aufweist; Cyano; $-S(O)_lR^{18}$ ($R^{18}$ bedeutet ein Alkyl mit 1 bis 20 Kohlenstoffatomen; Phenyl; oder ein Phenyl, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist; Carboxyl oder Alkoxycarbonyle, in welchen der Alkoxyrest 1 bis 8 Kohlenstoffatome aufweist und l 0, 1 oder 2 ist), oder

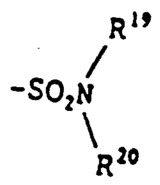

($R^{19}$ und $R^{20}$ sind gleich oder verschieden und jeder bedeutet Wasserstoff oder ein Alkyl mit 1 bis 20 Kohlenstoffatomen), worin X Sauerstoff oder Schwefel bedeutet und $R^9$ die Formel:

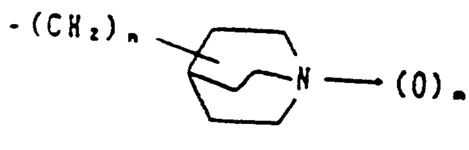

bedeutet (m ist 0 oder 1 und n ist 0, 1 oder 2),

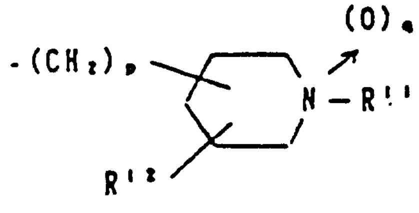

(p ist 0, 1 oder 2, q ist 0 oder 1, $R^{11}$ bedeutet ein Alkyl mit 1 bis 20 Kohlenstoffatomen; ein Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Phenoxyalxyl, das Phenoxymethyl, 2-Phenoxyethyl, 3-Phenoxypropyl oder 4-Phenoxybutyl ist; oder ein substituiertes Phenoxyalkyl, das Phenoxymethyl, 2-Phenoxyethyl, 3-Phenoxypropyl oder 4-Phenoxybutyl ist, welches durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist und worin $R^{12}$ Wasserstoff oder ein Alkoxy mit 1 bis 8 Kohlenstoffatomen bedeutet),

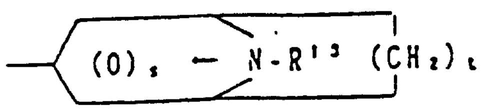

(s und t sind jeweils 0 oder 1 und $R^{13}$ bedeutet ein Alkyl mit 1 bis 20 Kohlenstoffatomen; ein Phenylalkyl, das Benzyl, 1- oder 2-Phenylethyl, 3-Phenylpropyl oder 4-Phenylbutyl ist; oder ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist),

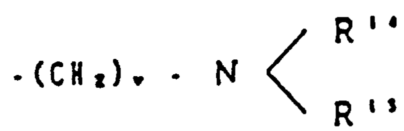

($R^{14}$ und $R^{15}$ sind gleich oder verschieden und jeder bedeutet Wasserstoff; ein Alkyl mit 1 bis 20 Kohlenstoffatomen; ein Phenylalkyl, das das Benzyl, 1- oder 2-Phenylethyl, 3-Phenylpropyl oder 4-Phenylbutyl ist; oder ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist, oder $R^{14}$ und $R^{15}$ sind aneinander gebunden um eine Gruppe zu bilden, die zusammen mit dem benachbarten Stickstoffatom einen heterocyclischen Ring bildet und v ist ein ganze Zahl von 1 bis 8), oder

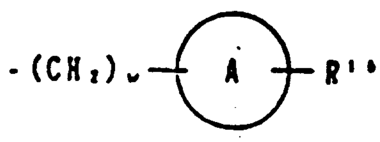

(Ring A bedeutet eine kondensierte oder nicht kondensierte Stickstoff enthaltende heterocyclische Ringruppe, die weiterhin Sauerstoff, Schwefel oder N-$R^{17}$ ($R^{17}$ bedeutet Wasserstoff oder ein Alkyl mit 1 bis 20 Kohlenstoffatomen) in dem Ring davon enthalten kann, $R^{16}$ bedeutet Wasserstoff; ein Alkyl mit 1 bis 20 Kohlenstoffatomen; ein Alkenyl mit 2 bis 20 Kohlenstoffatomen; ein Alkinyl mit 2 bis 20 Kohlenstoffatomen; ein Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; Amino; ein mono- oder di-Alkyl substituiertes Amino, worin der Alkylrest 1 bis 8 Kohlenstoffatome aufweist; oder ein Acylamino, das Alkanoylamino mit 2 bis 5 Kohlenstoffatomen ist und w ist 1, 2, 3 oder 4)).

2. Verbindung nach Anspruch 1, worin die Verbindung ausgewählt ist aus der Gruppe, bestehend aus 6-Chlor-4-methyl-N-(3-quinuclidinyl)-3,4-dihydro-2H-1,4-benzoxazin-8-carboxamid, (R)-6-Chlor-4-methyl-N-(3-quinuclidinyl)-3,4-dihydro-2H-1,4-benzoxazin-8-carboxamid, (S)-6-Chlor-4-methyl-N-(3-quinuclidinyl)-3,4-dihydro-2H-1,4-benzoxazin-8-carboxamid, 6-Chlor-2,2,4-trimethyl-N-(3-quinuclidinyl)-3,4-dihydro-2H-1,4-benzoxazin-8-carboxamid, (R)-6-Chlor-2,2,4-trimethyl-N-(3-quinuclidinyl)-3,4-dihydro-2H-1,4-benzoxozin-8- carboxamid, (S)-6-Chlor-2,2,4-trimethyl-N-(3-quinuclidinyl)-3,4-dihydro-2H-1,4-benzoxazin-8-carboxamid, 6-Fluor-4-methyl-N-(3-quinuclidinyl)-3,4-dihydro-2H-1,4-benzoxazin-8-carboxamid, 6-Chlor-N-(3-quinuclidinyl)-3,4-dihydro-2H-1,4-benzoxazin-8-carboxamid, 4-Acetyl-6-Chlor-N-(3-quinuclidinyl)-3,4-dihydro-2H-1,4-benzoxazin-8-carboxamid, 6-Chlor-4-methyl-N-(8-methyl-8-azobicyclo[3.2.1]oct-3-yl)-3,4-dihydro-2H-1,4-benzoxazin-8-carboxamid und pharmazeutisch verträgliche Salze davon.

3. Benzazinverbindung, ein geometrisches Isomer der Benzazinverbindung, ein optisches Isomer der Benzazinverbindung und ein pharmazeutisch verträgliches Salz der Benzazinverbindung, wobei die Benzazinverbindung durch die Formel (II) dargestellt ist:

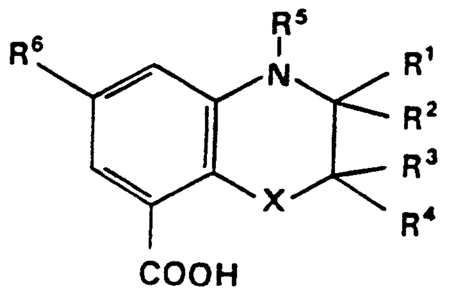

(II)

worin jedes Symbol die gleiche Bedeutung wie in Anspruch 1 definiert hat oder ein reaktives Derivat davon.

4. Pharmazeutische Zusammensetzung, umfassend eine Verbindung nach einem der vorhergehenden Ansprüche und einen pharmazeutisch verträgliches Träger-, Füll- oder Verdünnungsmittel.

5. Verwendung einer Verbindung nach einem der Ansprüche 1 bis 3 bei der Herstellung einer pharmazeutischen Formulierung zur Behandlung eines Zustandes der einen Antagonisten für 5-$HT_3$-Rezeptoren erfordert.

**Patentansprüche für folgenden Vertragsstaat : ES**

1. Verfahren zur Herstellung einer Benzazinverbindung, eines geometrischen Isomers der Benzazinverbindung, eines optischen Isomers der Benzazinverbindung und eines pharmazeutisch verträglichen Salzes der Benzazinverbindung, wobei die Benzazinverbindung durch die Formel (I) dargestellt ist:

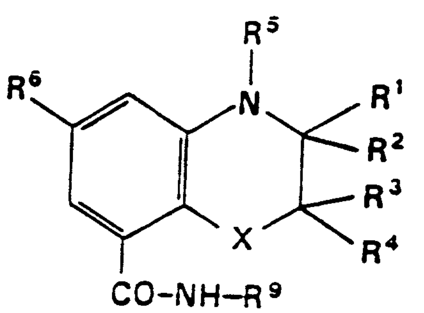

(I)

(worin $R^1$, $R^2$, $R^3$ und $R^4$ gleich oder verschieden sind, und jeweils Wasserstoff; ein Alkyl mit 1 bis 20 Kohlenstoffatomen; Phenyl; ein Phenyl, das mit 1 bis 3 Substituenten substituiert ist, die ausgewählt sind aus Halogenen,

Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonyle, in welchen der Alkoxyrest 1 bis 8 Kohlenstoffatome aufweist; ein Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Heteroaryl, das Pyridyl, Thienyl, Furyl, Imidazolyl, Pyrazolyl oder Pyrimidinyl ist; ein substituiertes Heteroaryl, das Pyridyl, Thienyl, Furyl, Imidazolyl, Pyrazolyl oder Pyrimidinyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Heteroarylalkyl, worin Heteroaryl Pyridyl, Thienyl, Furyl, Imidazolyl, Pyrazolyl oder Pyrimidinyl bedeutet und der Alkylrest 1 bis 8 Kohlenstoffatome aufweist; oder ein substituiertes Heteroarylalkyl, worin Heteroaryl Pyridyl, Thienyl, Furyl, Imidazolyl, Pyrazolyl oder Pyrimidinyl bedeutet, worin der Alykylrest 1 bis 8 Kohlenstoffatome aufweist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist, bedeutet; oder worin einer von $R^1$ und $R^2$ an einen von $R^3$ und $R^4$ gebunden ist, um eine Einfachbindung zu bilden, $R^5$ Wasserstoff; ein Alkyl mit 1 bis 20 Kohlenstoffatomen; ein Alkanoyl mit 2 bis 5 Kohlenstoffatomen; ein Aroyl das Benzoyl, Naphthoyl, Phenylacetyl, Phenylpropionyl oder Phenylbutyril; ein substituiertes Aroyl, das Benzoyl, Naphthoyl, Phenylacetyl, Phenylpropionyl oder Phenylbutyril ist, welches durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist;

ein Heteroaroyl, das 2- oder 3-Furoyl, 2- oder 3-Thienoyl, Nikotinoyl oder iso-Nikotinoyl ist; ein substituiertes Heteroaroyl, das 2- oder 3-Furoyl, 2- oder 3-Thienoyl, Nikotinoyl oder iso-Nikotinoyl ist, das durch 1 bis 3 Substituenten substituiert ist; ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Phenylalkyl das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; Carbamoyl; ein Carbamoyl, das durch 1 bis 2 Alkyle mit 1 bis 8 Kohlenstoffatomen substituiert ist; ein Aminoalkyl, worin der Alkylrest 1 bis 8 Kohlenstoffatome aufweist; oder ein mono- oder disubstituiertes Aminoalkyl, worin der Aminorest durch Methyl substituiert worden ist und der Alkylrest 1 bis 8 Kohlenstoffatome aufweist, bedeutet; oder worin $R^5$ an einen von $R^1$ oder $R^2$ gebunden sein kann, um eine Einfachbindung zu bilden, $R^6$ Wasserstoff; ein Halogen; ein Alkyl mit 1 bis 20 Kohlenstoffatomen; ein Alkoxy mit 1 bis 8 Kohlenstoffatomen; Amino; ein Acylamino, das Alkanoylamino mit 2 bis 5 Kohlenstoffatomen ist; ein mono- oder di-Alkyl substituiertes Amino, worin der Alkylrest 1 bis 8 Kohlenstoffatome aufweist; eine Hydroxylgruppe; eine geschützte Hydroxylgruppe; Nitro; ein Haloalkyl, worin der Alkylrest 1 bis 4 Kohlenstoffatome aufweist; Cyano; $-S(O)_lR^{18}$ ($R^{18}$ bedeutet ein Alkyl mit 1 bis 20 Kohlenstoffatomen; Phenyl; oder ein Phenyl, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist; Carboxyl oder Alkoxycarbonyle, in welchen der Alkoxyrest 1 bis 8 Kohlenstoffatome aufweist und l 0, 1 oder 2 ist); oder

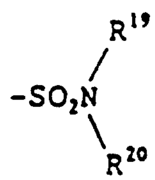

($R^{19}$ und $R^{20}$ sind gleich oder verschieden und jeder bedeutet Wasserstoff oder ein Alkyl mit 1 bis 20 Kohlenstoffatomen), worin X Sauerstoff oder Schwefel und $R^9$ die Formel:

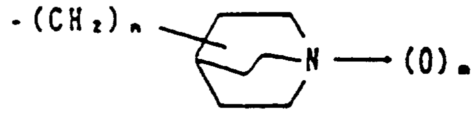

bedeutet (m ist 0 oder 1 und n ist 0, 1 oder 2),

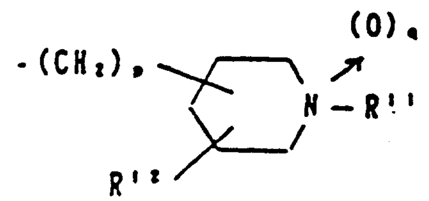

(p ist 0, 1 oder 2, q ist 0 oder 1, $R^{11}$ bedeutet ein Alkyl mit 1 bis 20 Kohlenstoffatomen; ein Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; ein Phenoxyalkyl, das Phenoxymethyl, 2-Phenoxyethyl, 3-Phenoxypropyl oder 4-Phenoxybutyl ist; oder ein substituiertes Phenoxyalkyl, das Phenoxymethyl, 2-Phenoxyethyl, 3-Phenoxypropyl oder 4-Phenoxybutyl ist, welches durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist und worin $R^{12}$ Wasserstoff oder ein Alkoxy mit 1 bis 8 Kohlenstoffatomen bedeutet),

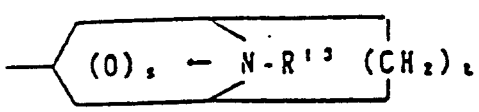

(s und t sind jeweils 0 oder 1 und $R^{13}$ bedeutet ein Alkyl mit 1 bis 20 Kohlenstoffatomen; ein Phenylalkyl, das Benzyl, 1- oder 2-Phenylethyl, 3-Phenylpropyl oder 4-Phenylbutyl ist; oder ein substituiertes Phenylalkyl, das Benzyl, 1-oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist),

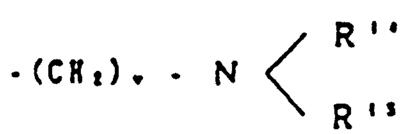

($R^{14}$ und $R^{15}$ sind gleich oder verschieden und jeder bedeutet Wasserstoff; ein Alkyl mit 1 bis 20 Kohlenstoffatomen; ein Phenylalkyl, das Benzyl, 1- oder 2-Phenylethyl, 3-Phenylpropyl oder 4-Phenylbutyl ist, oder ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und

Alkoxycarbonylen, in welchen der Alkoxyrest 1 bis 8 Kohlenstoffatome aufweist, oder $R^{14}$ und $R^{15}$ sind aneinander gebunden um eine Gruppe zu bilden, die zusammen mit dem benachbarten Stickstoffatom einen heterocyclischen Ring bildet und v ist ein ganze Zahl von 1 bis 8); oder

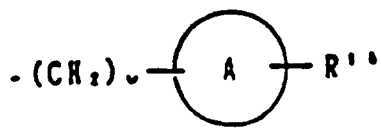

(Ring A bedeutet eine kondensierte oder nicht kondensierte Stickstoff enthaltende heterocyclische Ringruppe, die weiterhin Sauerstoff, Schwefel oder N-$R^{17}$ ($R^{17}$ bedeutet Wasserstoff oder ein Alkyl mit 1 bis 20 Kohlenstoffatomen) in dem Ring davon enthalten kann, $R^{16}$ bedeutet Wasserstoff; ein Alkyl mit 1 bis 20 Kohlenstoffatomen; ein Alkenyl mit 2 bis 20 Kohlenstoffatomen; ein Alkinyl mit 2 bis 20 Kohlenstoffatomen; Phenylalkyl, das Benzyl, 1- oder 2-Phenylethyl, 3-Phenylpropyl oder 4-Phenylbutyl ist; ein substituiertes Phenylalkyl, das Benzyl, 1- oder 2-Phenylethyl, 3-Phenylpropyl oder 4-Phenylbutyl ist, das durch 1 bis 3 Substituenten substituiert ist, ausgewählt aus Halogenen, Alkoxylen mit 1 bis 8 Kohlenstoffatomen, Alkylen mit 1 bis 20 Kohlenstoffatomen, Nitro, Amino, Haloalkyl, worin der Alkylrest 1 bis 4 Kohlenstoffatome aufweist, Carboxy und Alkoxycarbonylen, in welchen der Alkoxyrest 1 bis 8 Kohlenstoffatome aufweist; Amino; ein mono- oder di-Alkyl substituiertes Amino, worin der Alkylrest 1 bis 8 Kohlenstoffatome aufweist; oder ein Acylamino, das Alkanoylamino mit 2 bis 5 Kohlenstoffatomen ist und w ist 1, 2, 3 oder 4)), wobei das Verfahren das Umsetzen einer Benzazinverbindung, eines geometrischen Isomers der Benzazinverbindung, eines optischen Isomers der Benzazinverbindung und eines pharmazeutisch verträglichen Salzes der Benzazinverbindung, wobei die Benzazinverbindung durch die Formel (II) dargestellt ist:

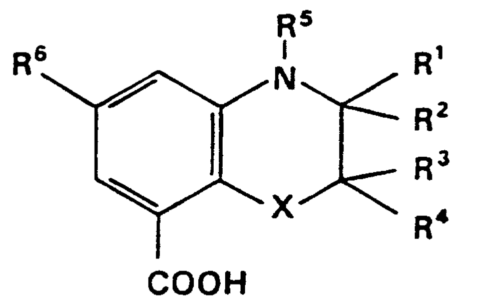

(II)

worin jedes Symbol die gleiche Bedeutung wie vorstehend definiert hat, oder eines reaktiven Derivats davon, mit einer Verbindung, die durch Formel (III) dargestellt ist:

$$R^9\text{-}NH_2$$

(worin $R^9$ die gleiche Bedeutung, wie vorstehend definiert, hat).

2. Verfahren nach Anspruch 1, worin die Verbindung ausgewählt wird aus der Gruppe, bestehend aus 6-Chlor-4-methyl-N-(2-quinuclidinyl)-3,4-dihydro-2H-1,4-benzoxazin-8-carboxamid, (R)-6-Chlor-4-methyl-N-(2-quinuclidinyl)-3,4-dihydro-2H-1,4-benzoxazin-8-carboxamid, (S)-6-Chlor-4-methyl-N-(2-quinuclidinyl)-3,4-dihydro-2H-1,4-benzoxazin-8-carboxamid, (R)-6-Chlor-2,2,4-trimethyl-N-(3-quinuclidinyl)-3,4-dihydro-2H-1,4-benzoxazin-8-carboxamid, (S)-6-Chlor-2,2,4-trimethyl-N-(3-quinuclidinyl)-3,4-dihydro-2H-1,4-benzoxazin-8-carboxamid, 6-Fluor-4-methyl-N-(2-quinuclidinyl)-3,4-dihydro-2H-1,4-benzoxazin-8-carboxamid, 6-Chlor-N-(3-quinuclidinyl)-3,4-dihydro-2H-1,4-benzoxazin-8-carboxamid, 4-Acetyl-6-Chlor-N-(2-quinuclidinyl)-3,4-dihydro-2H-1,4-benzoxazin-8-carboxamid, 6-Chlor-4-methyl-N-(8-methyl-8-azobicyclo[3.2.1]oct-3-yl)-3,4-dihydro-2H-1,4-benzoxazin-8-carboxamid und pharmazeutisch verträgliche Salze davon.

3. Verfahren zur Herstellung einer Benzazinverbindung, eines geometrischen Isomers der Benzazinverbindung, eines optischen Isomers der Benzazinverbindung und eines pharmazeutisch verträglichen Salzes der Benzazinverbindung, wobei die Benzazinverbindung durch die Formel (II) dargestellt ist:

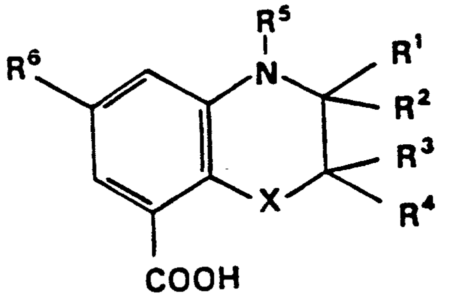

(II)

worin jedes Symbol die gleiche Bedeutung wie in Anspruch 1 definiert hat oder eines reaktiven Derivats davon,

worin die Verbindung unter Verwendung des nachstehend angegebenen Reaktionsweges erzeugt wird:

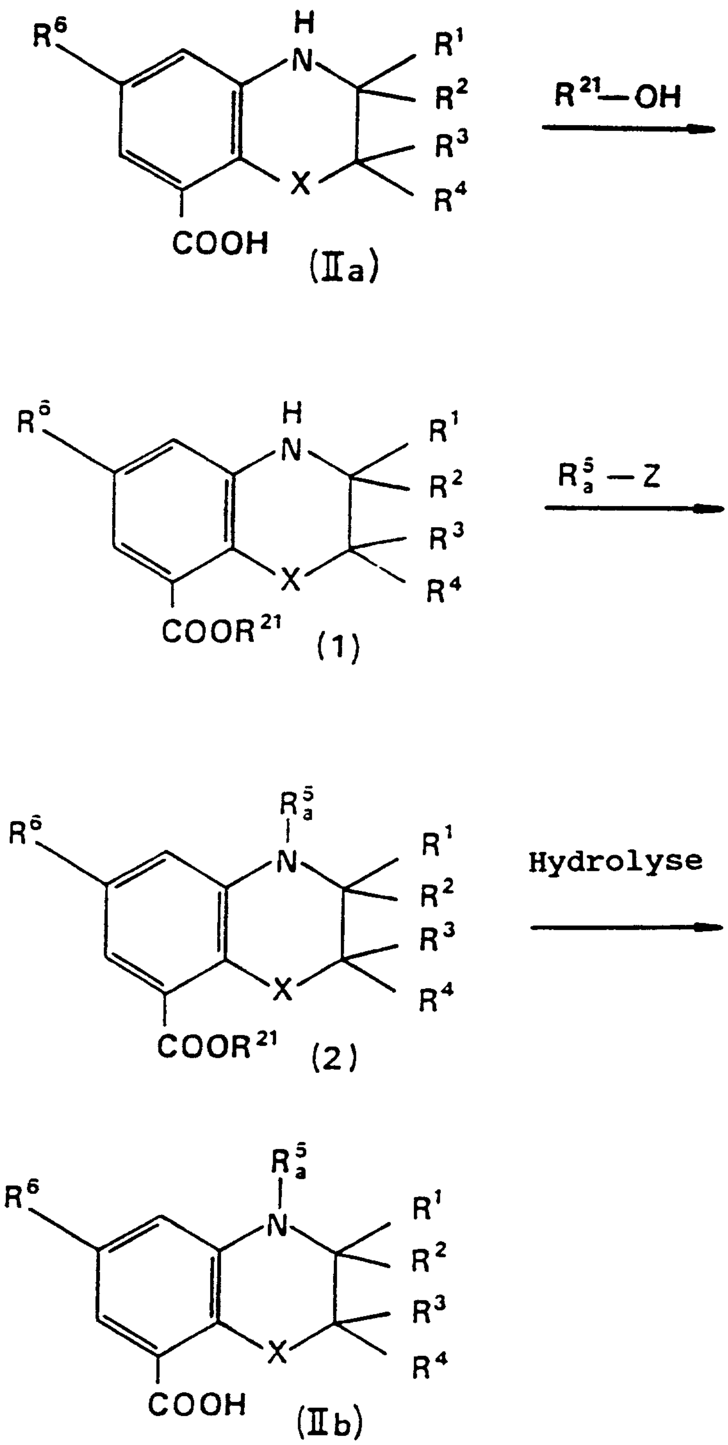


(IIa) (1) (2) (IIb)

4. Verfahren zur Herstellung einer Benzazinverbindung nach Anspruch 1, wobei das Verfahren das Umsetzen einer durch die Formel (II) dargestellten Verbindung:

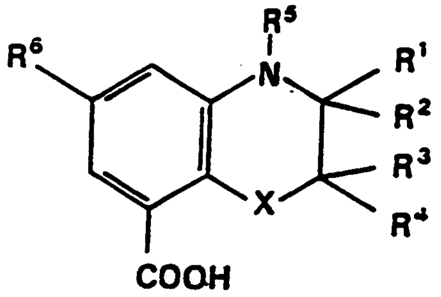

(II)

umfaßt, um die Benzazinverbindung bereitzustellen.

**Revendications**

**Revendications pour les Etats contractants suivants : DE, GB, FR, IT, NL, SE, CH, LI, BE, AT, LU, DK**

1. Dérivé de benzazine, isomère géométrique dudit dérivé de benzazine, isomère optique dudit dérivé de benzazine, et sel pharmaceutiquement acceptable dudit dérivé de benzazine, ledit dérivé de benzazine étant représente par la formule (I) :

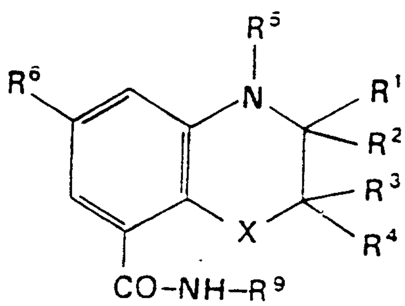

(I)

(dans laquelle $R^1$, $R^2$, $R^3$ et $R^4$ sont identiques ou différents et représentent chacun l'hydrogène ; un groupe alkyle ayant 1 à 20 atomes de carbone ; un groupe phényle ; un groupe phényle substitué portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénylalkyle qui consiste en un groupe benzyle, 1- ou 2-phényléthyle, 3-phénylpropyle ou 4-phénylbutyle, un groupe phénylalkyle substitué qui consiste en un groupe benzyle, 1- ou 2-phényléthyle, 3-phénylpropyle ou 4-phénylbutyle portant 1 à 3 substituants choisis entre les substituants halogéno, alkoxy ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroaryle qui est un groupe pyridyle, thiényle, furyle, imidazolyle, pyrazolyle ou pyrimidinyle ; un groupe hétéroaryle substitué qui est un groupe pyridyle, thiényle, furyle, imidazolyle, pyrazolyle ou pyrimidinyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroarylalkyle dans lequel le groupe hétéroaryle consiste en un groupe pyridyle, thiényle, furyle, imidazolyle, pyrazolyle ou pyrimidinyle et le groupement alkyle a 1 à 8 atomes de carbone ; ou un groupe hétéroarylalkyle substitué dans lequel le groupe hétéroaryle consiste en un groupe pyridyle, thiényle, furyle, imidazolyle, pyrazolyle ou pyrimidinyle et le groupement alkyle a 1 à 8 atomes de carbone portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; ou bien

un des groupes $R^1$ et $R^2$ est lié à un des groupes $R^3$ et $R^4$ formant une liaison simple ; $R^5$ représente l'hydrogène, un groupe alkyle ayant 1 à 20 atomes de carbone ; un groupe alcanoyle ayant 2 à 5 atomes de carbone ; un groupe aroyle qui consiste en un groupe benzoyle, naphtoyle, naphtoyle, phénylacétyle, phénylpropionyle ou phénylbutyryle ; un groupe aroyle substitué qui consiste enun groupe benzoyle, naphtoyle, phénylacétyle, phénylpropionyle ou phénylbutyryle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroaroyle qui consiste en un groupe 2- ou 3-furoyle, 2- ou 3-thiényle, nicotinoyle ou isonicotinoyle ; un groupe hétéroaroyle substitué qui consiste en un groupe 2- ou 3-furoyle, 2- ou 3-thiényle, nicotinoyle ou isonicotinoyle portant 1 à 3 substituants choisis encre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénylalkyle qui est un groupe benzyle, 1- ou 2-phényléthyle, 3-phénylpropyle ou 4-phénylbutyle ; un groupe phénylalkyle substitué qui est un groupe benzyle, 1- ou 2-phényléthyle, 3-phénylpropyle ou 4-phénylbutyle portant 1 à 3 substituants choisis entre des substituants halogéno, alkoxyls ayant 1 à 8 atomes de carbone, alkyle ayant 1 à 20 atomes de carbone, nitro amino, halogénalkyle dans lequel le groupement alkyle a 1 à 4 atomes de carbone, carboxy et alkoxycarbonyle dans lesquels le groupement alkoxy a 1 à 8 atomes le carbone ; un groupe carbamoyle, un groupe carbamoyle substitué avec un ou deux groupes alkyle ayant 1 à 8 atomes de carbone, un groupe aminoalkyle dans lequel le groupement alkyle a 1 à 8 atomes de carbone ; ou un groupe aminoalkyle, mono- ou disubstitué dans lequel le groupement amino a été substitué avec un groupe méthyle et le groupement alkyle a 1 à 8 atomes de carbone ; ou bien $R^5$ peut être lié à un des groupes $R^1$ et $R^2$ en formant une liaison simple ; $R^6$ représente l'hydrogène, un halogène ; un groupe alkyle ayant 1 à 20 atomes de carbone ; un groupe alkoxy ayant 1 à 8 atomes de carbone ; un groupe amino ; un groupe acylamino qui consiste en un groupe alcanoylamino ayant 2 à 5 atomes de carbone ; un groupe amino à substituant mono- ou dialkyle dans lequel le groupement alkyle a 1 à 8 atomes de carbone ; un groupe hydroxyle ; un groupe hydroxyle protégé, un groupe nitro, un groupe halogénalkyle dans lequel le groupe alkyle a 1 à 4 atomes de carbone ; un groupe cyano ; un groupe -S(O), $\ell$, $R^{18}$ ($R^{18}$ représente un groupe alkyle ayant 1 à 20 atomes de carbone, un groupe phényle ou un groupe phényle portant 1 à 3 substituants choisis entre des substituants halogéno, alkoxyle ayant 1 à 8 atomes de carbone ; alkyle ayant 1 à 20 atomes de carbone ; nitro, amino, halogénalkyle dans lequel le groupement alkyle a 1 à 4 atomes de carbone ; carboxyle ou alkoxycarbonyle dans lesquels le groupement alkoxy a 1 à 8 atomes de carbone, et $\ell$ est égal à 0, 1 ou 2), ou un groupe

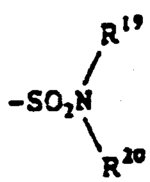

($R^{19}$ et $R^{20}$ sont identiques ou différents et représentent chacun l'hydrogène ou un groupe alkyle ayant 1 à 20 atomes de carbone) ; X représente l'oxygène ou le soufre ; et $R^9$ répond à la formule :

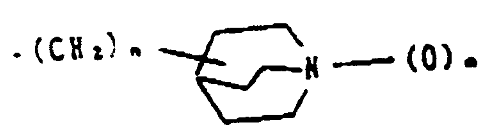

(m est égal à 0 ou 1 et n est égal à 0, 1 ou 2),

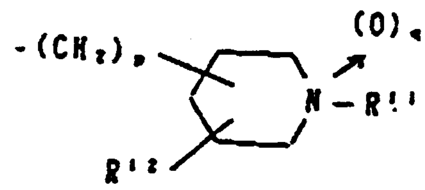

(p est égal à 0, 1 ou 2, q est égal à 0 ou 1, $R^{11}$ représente un groupe alkyle ayant 1 à 20 atomes de carbone ; un groupe phénylalkyle qui consiste en un groupe benzyle ou 2-phényléthyle, 3-phénylpropyle ou 4-phénylpropyle ; un groupe phénylalkyle substitué qui consiste en un groupe benzyle, 1- ou 2-phénylbutyle, 3-phénylpropyle, 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénoxyalkyle qui consiste en un groupe phénoxyméthyle, 2-phénoxyéthyle, 3-phénoxypropyle ou 4-phénoxybutyle ; ou un groupe phénoxyalkyle substitué qui consiste en un groupe phénoxyméthyle, 2-phénoxyéthyle, 3-phénoxypropyle ou 4-phénoxybutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lesquels le groupement alkyle a 1 à 4 atomes de carbone, carboxy et alkoxycarbonyle dans lesquels le groupement alkoxy a 1 à 8 atomes de carbone ; et $R^{12}$ représente l'hydrogène ou un groupe alkoxy ayant 1 à 8 atomes de carbone),

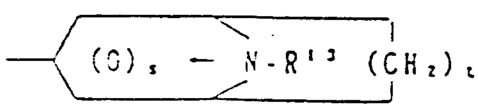

(s et t sons chacun égaux à 0 ou 1, et $R^{13}$ représente un groupe alkyle ayant 1 à 20 atomes de carbone ; un groupe phénylalkyle qui consiste en un groupe benzyle, 1- ou 2-phényléthyle, 3-phénylpropyle ou 4-phénylbutyle ; ou un groupe phénylalkyle substitué qui consiste en un groupe benzyle, 1- ou 2-phényléthyle, 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lesquels le groupement alkyle a 1 à 4 atomes de carbone, carboxy et alkoxycarbonyle dans lequel le groupement alkoxy a 1 à 8 atomes de carbone,

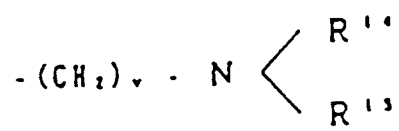

($R^{14}$ et $R^{15}$ sont identiques ou différents et représentent chacun l'hydrogène ; un groupe alkyle ayant 1 à 20 atomes de carbone ; un groupe phénylalkyle qui consiste en un groupe benzyle, 1- ou 2-phényléthyle, 3-phénylpropyle ou 4-phénylbutyle ; ou un groupe phénylalkyle substitué qui consiste en un groupe benzyle, 1- ou 2-phényléthyle, 3-phénylpropyle ou 4-phénylbutyle, portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; ou bien $R^{14}$ et $R^{15}$ sont liés l'un à l'autre en formant un groupe qui constitue un noyau hétérocyclique en coopération avec l'atome d'azote adjacent, et v est un nombre entier de 1 à 8), ou

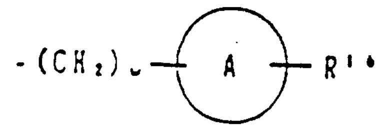

(le noyau A représente un groupe cyclique hétérocyclique azoté, condensé ou non-condensé), qui peut contenir en outre de l'oxygène, du soufre ou un groupe N-$R^{17}$ ($R^{17}$ représente l'hydrogène ou un groupe alkyle ayant 1 à 20 atomes de carbone) dans son noyau, $R^{16}$ représente l'hydrogène, un groupe alkyle ayant 1 à 20 atomes de carbone ; un groupe alcényle ayant 2 à 20 atomes de carbone ; un groupe alcynyle ayant 2 à 20 atomes de carbone, un groupe phénylalkyle qui consiste en un groupe benzyle, 1- ou 2-phényléthyle, 3-phénylpropyle ou 4-phénylbutyle ; un groupe phénylalkyle substitué qui consiste en un groupe benzyle, 1- ou 2-phényléthyle, 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe amino, un groupe amino à substituant mono- ou dialkyle dans lequel le groupement alkyle a 1 à 8 atomes de carbone ; ou un groupe acylamino qui consiste en un groupe alcanoylamino ayant 2 à 5 atomes de carbone, et W est égal à 1, 2, 3 ou 4.

**2.** Composé suivant la revendication 1, qui est choisi dans le groupe consistant en 6-chloro-4-méthyl-N-(3-quinuclidinyl)-3,4-dihydro-2H-1,4-benzoxazine-8-carboxamide, (R)-6-chloro-4-méthyl-N-(3-quinuclidinyl)-3,4-dihydro-2H-1,4-benzoxazine-8-carboxamide, (S)-6-chloro-4-méthyl-N-(3-quinuclidinyl)3,4-dihydro-2H-1,4-benzoxazine-8-carboxamide, 6-chloro-2,2,4-triméthyl-N-(3-quinuclidinyl)-3,4-dihydro-2H-1,4-benzoxazine-8-carboxamide, (R)-6-chloro-2,2,4-triméthyl-N-(3-quinuclidinyl)-3,4-dihydro-2H-1,4-benzoxazine-8-carboxamide, (S)-6-chloro-2,2,4-triméthyl-N-(3-quinuclidinyl)-3,4-dihydro-2H-1,4-benzoxazine-8-carboxamide,6-fluoro-4-méthyl-N-(3-quinuclidinyl)-3,4-dihydro-2H-1,4-benzoxazine-8-carboxamide, 6-chloro-N-(3-quinuclidinyl)-3,4-dihydro-2H-1,4-benzoxazine-8-carboxamide, 4-acétyl-6-chloro-N-(3-quinuclidinyl)-3,4-dihydro-2H-1,4-benzoxazine-8-carboxamide, 6-chloro-4-méthyl-N-(8-méthyl-8-azobicyclo[3.2.1]oct-3-yl)-3,4-dihydro-2H-1,4-benzoxazine-8-carboxamide et leurs sels pharmaceutiquement acceptables.

**3.** Dérivé de benzazine, isomère géométrique dudit dérivé de benzazine, isomère optique dudit dérivé de benzazine, et sel pharmaceutiquement acceptable dudit dérivé de benzazine, ledit dérivé de benzazine étant représenté par la formule (II) :

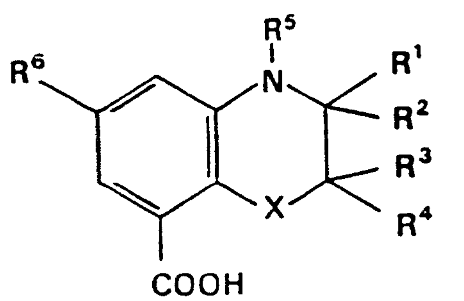

(II)

dans laquelle chaque symbole a la même signification que celle définie dans la revendication 1, ou un de ses dérivés réactifs.

**4.** Composition pharmaceutique comprenant un composé suivant l'une quelconque des revendications précédentes et un support, excipient ou diluant pharmaceutiquement acceptable.

**5.** Utilisation d'un composé suivant l'une quelconque des revendications 1 à 3 dans la préparation d'une formulation pharmaceutique pour le traitement d'une affection qui requiert un antagoniste des récepteurs 5-$HT_3$.

**Revendications pour l'Etat contractant suivant : ES**

1. Procédé de préparation d'un dérivé de benzazine, d'un isomère géométrique dudit dérivé de benzazine, d'un isomère optique dudit dérivé de benzazine, ou d'un sel pharmaceutiquement acceptable dudit dérivé de benzazine, ledit dérivé de benzazine étant représenté par la formule (I) :

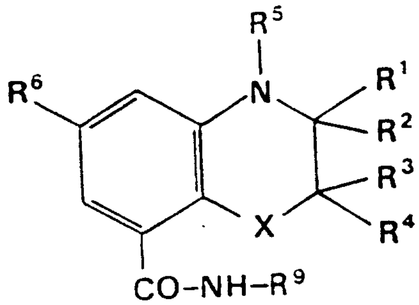

(I)

(dans laquelle $R^1$, $R^2$, $R^3$ et $R^4$ sont identiques ou différents et représentent chacun l'hydrogène ; un groupe alkyle ayant 1 à 20 atomes de carbone ; un groupe phényle ; un groupe phényle substitué portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénylalkyle qui consiste en un groupe benzyle, 1- ou 2-phényléthyle, 3-phénylpropyle ou 4-phénylbutyle, un groupe phénylalkyle substitué qui consiste en un groupe benzyle, 1- ou 2-phényléthyle, 3-phénylpropyle ou 4-phénylbutyle portant 1 à 3 substituants choisis entre les substituants halogéno, alkoxy ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroaryle qui est un groupe pyridyle, thiényle, furyle, imidazolyle, pyrazolyle ou pyrimidinyle ; un groupe hétéroaryle substitué qui est un groupe pyridyle, thiényle, furyle, imidazolyle, pyrazolyle ou pyrimidinyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroarylalkyle dans lequel le groupe hétéroaryle consiste en un groupe pyridyle, thiényle, furyle, imidazolyle, pyrazolyle ou pyrimidinyle et le groupement alkyle a 1 à 8 atomes de carbone ; ou un groupe hétéroarylalkyle substitué dans lequel le groupe hétéroaryle consiste en un groupe pyridyle, thiényle, furyle, imidazolyle, pyrazolyle ou pyrimidinyle et le groupement alkyle a 1 à 8 atomes de carbone portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; ou bien un des groupes $R^1$ et $R^2$ est lié à un des groupes $R^3$ et $R^4$ formant une liaison simple ; $R^5$ représente l'hydrogène, un groupe alkyle ayant 1 à 20 atomes de carbone ; un groupe alcanoyle ayant 2 à 5 atomes de carbone ; un groupe aroyle qui consiste en un groupe benzoyle, naphtoyle, naphtoyle, phénylacétyle, phénylpropionyle ou phénylbutyryle ; un groupe aroyle substitué qui consiste enun groupe benzoyle, naphtoyle, phénylacétyle, phénylpropionyle ou phénylbutyryle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe hétéroaroyle qui consiste en un groupe 2- ou 3-furoyle, 2- ou 3-thiényle, nicotinoyle ou isonicotinoyle ; un groupe hétéroaroyle substitué qui congiste en un groupe 2- ou 3-furoyle, 2- ou 3-thiényle, nicotinoyle ou isonicotinoyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénylalkyle qui est un groupe benzyle, 1- ou 2-phényléthyle, 3-phénylpropyle ou 4-phénylbutyle ; un groupe phénylalkyle substitué qui est un groupe benzyle, 1- ou 2-phényléthyle, 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe carbamoyle, un groupe carbamoyle substitué avec un ou deux groupes alkyle ayant 1 à 8 atomes de carbone, un groupe aminoalkyle dans lequel le groupement alkyle a 1 à 8 atomes de carbone ; ou un groupe aminoalkyle, mono- ou disubstitué dans lequel le groupement amino a été substitué avec un groupe méthyle et le groupement alkyle a 1 à 8 atomes de carbone ; ou bien $R^5$ peut être lié à un des groupes $R^1$ et $R^2$ en formant une liaison simple ; $R^6$ représente l'hydrogène, un halogène ; un groupe alkyle ayant 1 à 20 atomes de carbone ; un groupe alkoxy ayant 1 à 8 atomes de carbone ; un groupe amino ; un groupe acylamino qui consiste en un groupe alcanoylamino ayant 2 à 5 atomes de carbone ; un groupe amino à substituant mono- ou dialkyle dans lequel le groupement alkyle a 1 à 8 atomes de carbone ; un groupe hydroxyle ; un groupe hydroxyle protégé, un groupe nitro, un groupe halogénalkyle dans lequel le groupe alkyle a 1 à 4 atomes de carbone ; un groupe cyano ; un groupe -S(O), $\ell$, $R^{18}$ ($R^{18}$ représente un groupe alkyle ayant 1 à 20 atomes de carbone, un groupe phényle ou un groupe phényle portant 1 à 3 substituants choisis entre des substituants halogéno, alkoxyle ayant 1 à 8 atomes de carbone ; alkyle ayant 1 à 20 atomes de carbone ; nitro, amino, halogénalkyle dans lesquels le groupement alkyle a 1 à 4 atomes de carbone ; carboxyle ou alkoxycarbonyle dans lesquels le groupement alkoxy a 1 à 8 atomes de carbone, et $\ell$ est égal à 0, 1 ou 2) ou un groupe

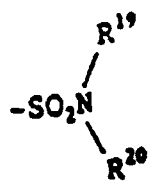

($R^{19}$ et $R^{20}$ sont identiques ou différents et représentent chacun l'hydrogène ou un groupe alkyle ayant 1 à 20 atomes de carbone) ; X représente l'oxygène ou le soufre ; et $R^9$ répond à la formule :

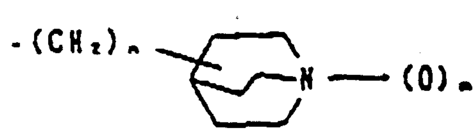

(m est égal à 0 ou 1 et n est égal à 0, 1 ou 2),

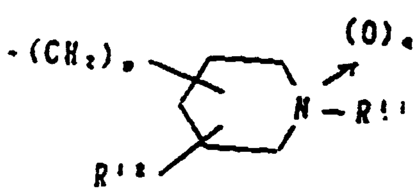

(p est égal à 0, 1 ou 2, q est égal à 0 ou 1, $R^{11}$ représente un groupe alkyle ayant 1 à 20 atomes de carbone ; un groupe un groupe alkyle ayant 1 à 20 atomes de carbone ; un groupe phénylalkyle qui consiste en un groupe benzyle 1- ou 2-phényléthyle, 3-phénylpropyle ou 4-phénylbutyle, un groupe phénylbutyle substitué qui consiste en un groupe benzyle, 1- ou 2-phényléthyle 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; un groupe phénoxyalkyle qui consiste en un groupe phénoxyméthyle, 2-phénoxyéthyle, 3-phénoxypropyle ou 4-phénoxybutyle ; ou un groupe phénoxyalkyle substitué qui consiste en un groupe phénoxyméthyle, 2-phénoxyéthyle, 3-phénoxypropyle ou 4-phénoxybutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; et $R^{12}$ représente

l'hydrogène ou un groupe alkoxy ayant 1 à 8 atomes de carbone),

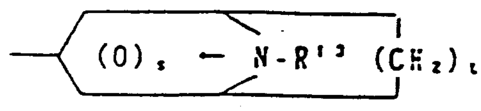

(s et t sont chacun égaux à 0 ou 1, et $R^{13}$ représente un groupe alkyle ayant 1 à 20 atomes de carbone ; un groupe phénylalkyle qui consiste en un groupe benzyle, 1- ou 2-phényléthyle, 3-phénylpropyle ou 4-phénylbutyle ; ou un groupe phénylalkyle substitué qui consiste en un groupe benzyle, 1- ou 2-phényléthyle, 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone,

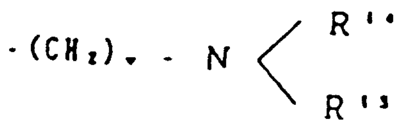

($R^{14}$ et $R^{15}$ sont identiques ou différents et représentent chacun l'hydrogène ; un groupe alkyle ayant 1 à 20 atomes de carbone ; un groupe phénylalkyle qui consiste en un groupe benzyle, 1- ou 2-phényléthyle, 3-phénylpropyle ou 4-phénylbutyle ; ou un groupe phénylalkyle substitué qui consiste en un groupe benzyle, 1- ou 2-phényléthyle, 3-phénylpropyle ou 4-phénylbutyle, portant 1 à 3 substituants choisis entre des substituants halogéno, alkoxyle ayant 1 à 8 atones de carbone, alkyle ayant 1 à 20 atomes de carbone, nitro, amino, halogénalkyle dans lequel le groupement alkyle a 1 à 4 atomes de carbone, carboxy et alkoxycarbonyle dans lesquels le groupement alkoxy a 1 à 8 atomes de carbone ; ou bien $R^{14}$ et $R^{15}$ sont liés l'un à l'autre en formant un groupe qui constitue un noyau hétérocyclique en coopération avec l'atome d'azote adjacent, et v est un nombre entier de 1 à 8), ou

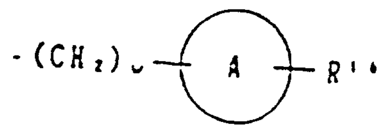

(le noyau A représente un noyau hétérocyclique azoté, condensé ou non condensé, qui peut contenir en outre de l'oxygène, du soufre ou un groupe N-$R^{17}$ ($R^{17}$ représente l'hydrogène ou un groupe alkyle ayant 1 à 20 atomes de carbone) dans son noyau, $R^{16}$ représente l'hydrogène, un groupe alkyle ayant 1 à 20 atomes de carbone ; un groupe alcényle ayant 2 à 20 atomes de carbone, un groupe alcynyle ayant 2 à 20 atomes de carbone ; un groupe phénylalkyle qui consiste en un groupe benzyle, 1- ou 2-phényléthyle, 3-phénylpropyle ou 4-phénylbutyle ; un groupe phénylalkyle substitué qui consiste en un groupe benzyle, 1- ou 2-phényléthyle, 3-phénylpropyle ou 4-phénylbutyle portant 1 à 3 substituants choisis entre des substituants halogéno, alkoxyle ayant 1 à 8 atomes de carbone, alkyle ayant 1 à 20 atomes de carbone, nitro, amino, halogénalkyle dans lesquels le groupement alkyle a 1 à 4 atomes de carbone, carboxy et alkoxycarbonyle dans lequel le groupement alkoxy a 1 à 8 atomes de carbone ; un groupe amino, un groupe amino à substituant mono- ou dialkyle dans lequel le groupement alkyle a 1 à 8 atomes de carbone ; ou un groupe acylamino qui consiste en un groupe alcanoylamino ayant 2 à 5 atomes de carbone, et W est égal à 1, 2, 3 ou 4), procédé qui comprend la réaction d'un dérivé de benzazine, un isomètre géométrique dudit dérivé de benzazine, d'un isomère optique dudit dérivé de benzazine ou d'un sel pharmaceutiquement acceptable dudit

dérivé de benzazine, ledit dérivé de benzazine étant représenté par la formule (II)

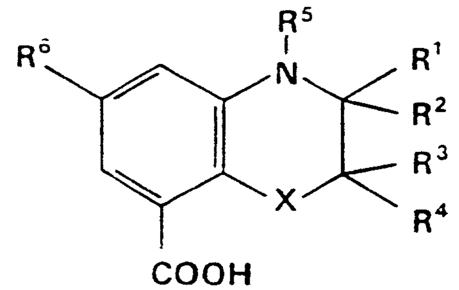

(II)

dans laquelle chaque symbole a la même définition que celle indiquée ci-dessus, ou d'un de leurs dérivés réactifs, avec un composé représenté par la formule (III)

$$R^9\text{-}NH_2$$

(dans laquelle $R^9$ répond à la définition précitée).

2. Procédé suivant la revendication 1, dans lequel le composé est choisi dans le groupe consistant en en 6-chloro-4-méthyl-N-(3-quinuclidinyl)-3,4-dihydro-2H-1,4-benzoxazine-8-carboxamide, (R)-6-chloro-4-méthyl-N-(3-quinuclidinyl)-3,4-dihydro-2H-1,4-benzoxazine-8-carboxamide, (S)-6-chloro-4-méthyl-N-(3-quinuclidinyl)-3,4-dihydro-2H-1,4-benzoxazine-8-carboxamide, 6-chloro-2,2,4-triméthyl-N-(3-quinuclidinyl)-3,4-dihydro-2H-1,4-benzoxazine-8-carboxamide, (R)-6-chloro-2,2,4-triméthyl-N-(3-quinuclidinyl)-3,4-dihydro-2H-1,4-benzoxazine-8-carboxamide, (S)-6-chloro-2,2,4-triméthyl-N-(3-quinuclidinyl)-3,4-dihydro-2H-1,4-benzoxazine-8-carboxamide, 6-fluoro-4-méthyl-N-(3-quinuclidinyl)-3,4-dihydro-2H-1,4-benzoxazine-8-carboxamide, 6-chloro-N-(3-quinuclidinyl)-3,4-dihydro-2H-1,4-benzoxazine-8-carboxamide, 4-acétyl-6-chloro-N-(3-quinuclidinyl)-3,4-dihydro-2H-1,4-benzoxazine-8-carboxamide, 6-chloro-4-méthyl-N-(8-méthyl-8-azobicyclo[3.2.1]oct-3-yl)-3,4-dihydro-2H-1,4-benzoxazine-8-carboxamide et leurs sels pharmaceutiquement acceptables.

3. Procédé de préparation d'un dérivé de benzazine, d'un isomère géométrique dudit dérivé de benzazine, d'un isomère optique dudit dérivé de benzazine ou d'un sel pharmaceutiquement acceptable dudit dérivé de benzazine, ledit dérivé de benzazine étant représenté par la formule (II) :

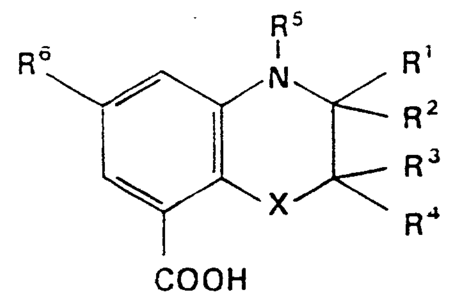

(II)

dans laquelle chaque symbole répond à la définition mentionnée dans la revendication 1, ou d'un de ses dérivés

réactifs, dans lequel ledit composé est produit en utilisant la voie réactionnelle indiquée ci-dessous :

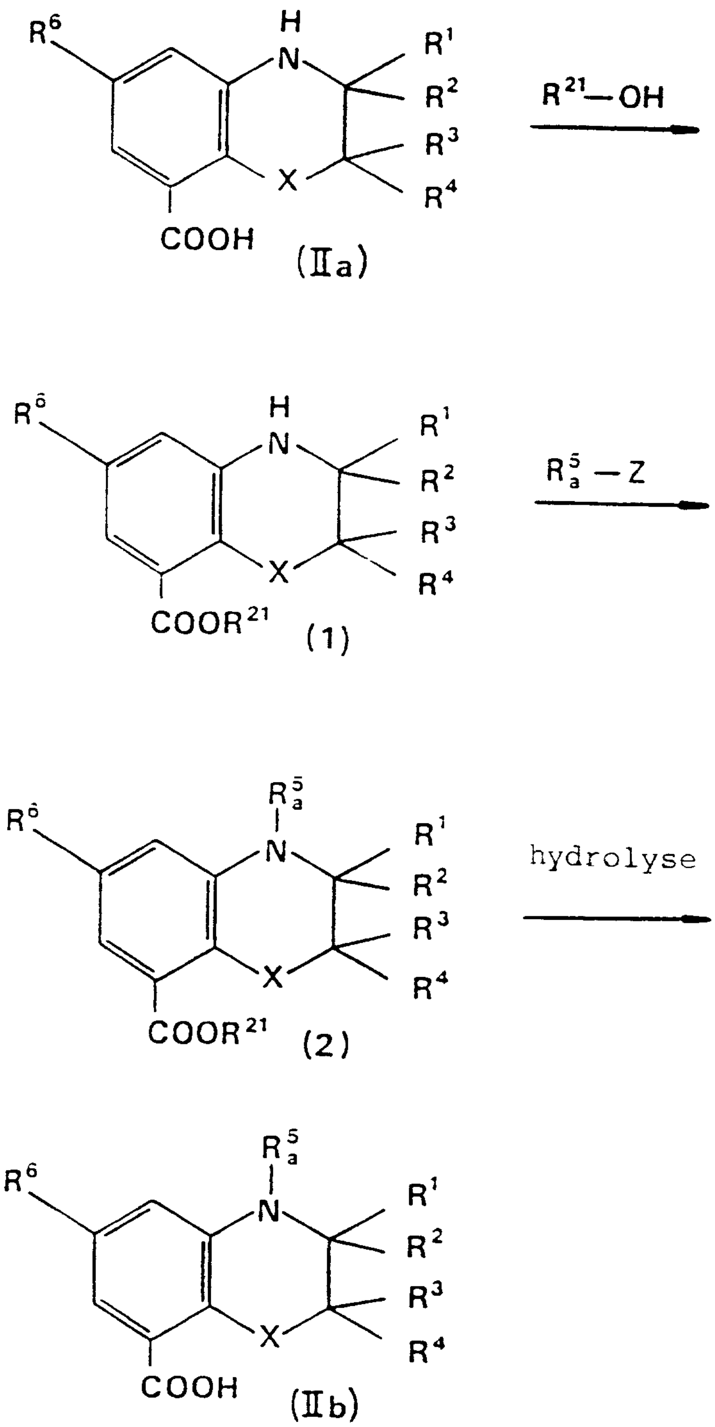


(IIa) (1) (2) (IIb)

4. Procédé de préparation d'un dérivé de benzazine répondant à la définition suivant la revendication 1, procédé qui comprend la réaction du composé représenté par la formule (II) :

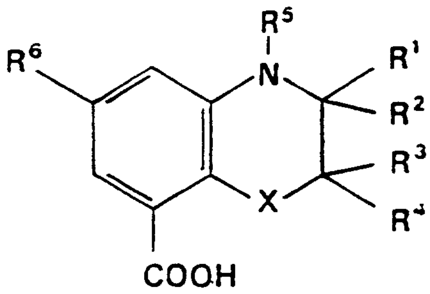

(II)

pour produire ledit dérivé de benzazine.